# EUROPEAN PATENT SPECIFICATION

(11) **EP 3 408 384 B1**
(45) Date of publication and mention of the grant of the patent: **13.11.2019**
(21) Application number: 17702333.0
(22) Date of filing: 26.01.2017
(51) Int. Cl.: C12N 9/04

(54) **MUTATED CELLOBIOSE DEHYDROGENASE WITH MODIFIED SUBSTRATE SPECIFICITY**
MUTIERTE CELLOBIOSE-DEHYDROGENASE MIT MODIFIZIERTER SUBSTRATSPEZIFITÄT
DÉSHYDROGÉNASE DE CELLOBIOSE MUTÉE DOTÉE D'UNE SPÉCIFICITÉ DE SUBSTRAT MODIFIÉE

(30) Priority: 26.01.2016 EP 16152770
(43) Date of publication of application: 05.12.2018
(73) Proprietor: DirectSens GmbH, 3400 Klosterneuburg (AT)
(72) Inventor: SYGMUND, Christoph, 3400 Klosterneuburg (AT); LUDWIG, Roland, 1220 Vienna (AT); STOICA, Leonard, 1220 Vienna (AT)
(74) Representative: Sonn & Partner Patentanwälte
(86) International application number: PCT/EP2017/051577
(87) International publication number: WO 2017/129637

(56) References cited:
- EP-A1- 2 223 936
- EP-A1- 2 636 733

### SEQUENCE LISTING

<110> Directsens GmbH
<120> Mutated cellobiose dehydrogenase with modified substrate
<130> r70421
<150> EP16152770.0
   <151> 2016-01-26
<160> 12
<170> PatentIn version 3.5
<210> 1
   <211> 831
   <212> PRT
   <213> Chaetomium atrobrunneum
<400> 1
   Met Arg Pro Ser Ser Arg Phe Val Gly Ala Leu Ala Ala Ala Ala Ser
   1 5 10 15
   Phe Leu Pro Ser Ala Leu Ala Gln Asn Asn Ala Ala Val Thr Phe Thr
   20 25 30
   Asp Pro Asp Thr Gly Ile Val Phe Asn Ser Trp Gly Leu Ala Asn Gly
   35 40 45
   Ala Pro Gln Thr Gln Gly Gly Phe Thr Phe Gly Val Ala Leu Pro Ser
   50 55 60
   Asp Ala Leu Thr Thr Asp Ala Thr Glu Phe Ile Gly Tyr Leu Glu Cys
   65 70 75 80
   Ala Ser Ala Asp Asn Gln Gly Trp Cys Gly Val Ser Met Gly Gly Pro
   85 90 95
   Met Thr Asn Ser Leu Leu Ile Thr Ala Trp Pro His Glu Asp Asn Val
   100 105 110
   Tyr Thr Ser Leu Arg Phe Ala Thr Gly Tyr Ala Met Pro Asp Val Tyr
   115 120 125
   Ser Gly Asp Ala Thr Ile Thr Gln Ile Ser Ser Ser Ile Asn Ala Thr
   130 135 140
   His Phe Lys Leu Ile Phe Arg Cys Gln Asn Cys Leu Gln Trp Thr His
   145 150 155 160
   Asp Gly Ala Ser Gly Gly Ala Ser Thr Ser Ala Gly Val Leu Val Leu
                   165                 170                 175
   Gly Trp Val Gln Ala Phe Pro Ser Pro Gly Asn Pro Thr Cys Pro Asp
               180                 185                 190
   Gln Ile Thr Leu Glu Gln His Asn Asn Gly Met Gly Ile Trp Gly Ala
           195                 200                 205
   Val Met Asp Ser Asn Val Ala Asn Pro Ser Tyr Thr Glu Trp Ala Ala
       210                 215                 220
   Gln Ala Thr Lys Thr Val Glu Ala Glu Cys Asp Gly Pro Ser Glu Thr
   225                 230                 235                 240
   Asp Ile Val Gly Val Pro Val Pro Thr Gly Thr Thr Phe Asp Tyr Ile
                   245                 250                 255
   Val Val Gly Gly Gly Ala Gly Gly Ile Pro Thr Ala Asp Lys Leu Ser
               260                 265                 270
   Glu Ala Gly Lys Ser Val Leu Leu Ile Glu Lys Gly Ile Ala Ser Thr
           275                 280                 285
   Ala Glu His Gly Gly Thr Leu Gly Pro Glu Trp Leu Glu Gly Asn Asp
       290                 295                 300
   Leu Thr Arg Phe Asp Val Pro Gly Leu Cys Asn Gln Ile Trp Val Asp
   305                 310                 315                 320
   Ser Lys Gly Ile Ala Cys Glu Asp Thr Asp Gln Met Ala Gly Cys Val
                   325                 330                 335
   Leu Gly Gly Gly Thr Ala Val Asn Ala Gly Leu Trp Phe Lys Pro Tyr
               340                 345                 350
   Ser Leu Asp Trp Asp Tyr Leu Phe Pro Ser Gly Trp Lys Tyr Arg Asp
           355                 360                 365
   Ile Gln Ala Ala Ile Gly Arg Val Phe Ser Arg Ile Pro Gly Thr Asp
       370                 375                 380
   Ala Pro Ser Thr Asp Gly Lys Arg Tyr Tyr Gln Gln Gly Phe Asp Val
   385                 390                 395                 400
   Leu Ala Gly Gly Leu Ser Ala Gly Gly Trp Asn Lys Val Thr Ala Asn
                   405                 410                 415
   Ser Ser Pro Asp Lys Lys Asn Arg Thr Phe Ser Asn Ala Pro Phe Met
               420                 425                 430
   Phe Ser Gly Gly Glu Arg Gly Gly Pro Leu Ala Thr Tyr Leu Thr Ser
           435                 440                 445
   Ala Lys Lys Arg Ser Asn Phe Asn Leu Trp Leu Asn Thr Ser Val Lys
       450                 455                 460
   Arg Val Ile Arg Glu Gly Gly His Val Thr Gly Val Glu Val Glu Pro
   465                 470                 475                 480
   Phe Arg Thr Gly Gly Tyr Gln Gly Ile Val Asn Val Thr Ala Val Ser
                   485                 490                 495
   Gly Arg Val Val Leu Ser Ala Gly Thr Phe Gly Ser Ala Lys Ile Leu
               500                 505                 510
   Leu Arg Gly Gly Ile Gly Pro Ala Asp Gln Leu Glu Val Val Lys Ala
           515                 520                 525
   Ser Lys Ile Asp Gly Pro Thr Met Ile Ser Asn Ala Ser Trp Ile Pro
       530                 535                 540
   Leu Pro Val Gly Tyr Asn Leu Asp Asp His Leu Asn Thr Asp Thr Val
   545                 550                 555                 560
   Ile Thr His Pro Asp Val Ala Phe Tyr Asp Phe Tyr Glu Ala Trp Asn
                   565                 570                 575
   Thr Pro Ile Glu Ala Asp Lys Asn Ser Tyr Leu Ser Ser Arg Thr Gly
               580                 585                 590
   Ile Leu Ala Gln Ala Ala Pro Asn Ile Gly Pro Met Met Trp Glu Glu
           595                 600                 605
   Ile Lys Gly Ala Asp Gly Ile Val Arg Gln Leu Gln Trp Thr Ala Arg
       610                 615                 620
   Val Glu Gly Ser Phe Asp Thr Pro Asn Gly Gln Ala Met Thr Ile Ser
   625                 630                 635                 640
   Gln Tyr Leu Gly Arg Gly Ala Thr Ser Arg Gly Arg Met Thr Ile Thr
                   645                 650                 655
   Pro Ser Leu Thr Thr Val Val Ser Asp Val Pro Tyr Leu Lys Asp Pro
               660                 665                 670
   Asn Asp Lys Glu Ala Val Ile Gln Gly Ile Val Asn Leu Gln Asn Ala
   675 680 685
   Leu Lys Asn Val Ala Gly Leu Thr Trp Thr Tyr Pro Asn Ser Ser Ile
   690 695 700
   Thr Pro Arg Glu Tyr Val Asp Asn Met Val Val Ser Pro Ser Asn Arg
   705 710 715 720
   Arg Ala Asn His Trp Met Gly Thr Ala Lys Ile Gly Thr Asp Asp Gly
   725 730 735
   Arg Leu Ala Gly Gly Ser Ala Val Val Asp Leu Asn Thr Lys Val Tyr
   740 745 750
   Gly Thr Asp Asn Leu Phe Val Val Asp Ala Ser Ile Phe Pro Gly Thr
   755 760 765
   Pro Thr Thr Asn Pro Ser Ala Tyr Ile Val Thr Ala Ala Glu His Ala
   770 775 780
   Ser Gln Arg Ile Leu Gly Leu Ala Ala Pro Lys Pro Val Gly Lys Trp
   785 790 795 800
   Gly Gln Cys Gly Gly Arg Gln Trp Thr Gly Ser Phe Gln Cys Val Ser
   805 810 815
   Gly Thr Lys Cys Glu Val Val Asn Glu Trp Tyr Ser Gln Cys Leu
   820 825 830
<210> 2
   <211> 845
   <212> PRT
   <213> Hypoxylon haematostroma
<400> 2
   Met Gly Arg Leu Gly Ser Leu Ala Lys Leu Leu Leu Ala Val Gly Leu
   1 5 10 15
   Asn Val Gln Gln Cys Phe Gly Gln Asn Gly Pro Pro Thr Pro Tyr Thr
   20 25 30
   Asp Ser Glu Thr Gly Ile Thr Phe Ala Thr Trp Ser Val Pro Asp Arg
   35 40 45
   Ala Glu Gly Gly Asn Gly Leu Ala Pro Trp Gly Gly Leu Thr Phe Gly
   50 55 60
   Val Ala Leu Pro Glu Asn Ala Leu Thr Thr Asp Ala Thr Glu Leu Ile
   65                  70                  75                  80
   Gly Tyr Leu Lys Cys Gly Ser Asn Gly Thr Thr Thr Asp Ala Trp Cys
                   85                  90                  95
   Gly Leu Ser Phe Gly Gly Pro Met Thr Asn Ser Leu Leu Leu Met Ala
               100                 105                 110
   Trp Pro His Glu Asp Glu Ile Leu Thr Ser Phe Arg Phe Ala Ser Gly
           115                 120                 125
   Tyr Thr Arg Pro Asp Leu Tyr Thr Gly Asp Ala Lys Leu Thr Gln Ile
       130                 135                 140
   Ser Ser Thr Ile Asp Lys Asp His Phe Thr Leu Ile Phe Arg Cys Gln
   145                 150                 155                 160
   Asn Cys Leu Ala Trp Asn Gln Asp Gly Ala Ser Gly Ser Ala Ser Thr
                   165                 170                 175
   Ser Ala Gly Ser Leu Ile Leu Gly Trp Ala Ser Ala Leu Arg Ala Pro
               180                 185                 190
   Thr Asn Ala Gly Cys Pro Ala Glu Ile Asn Phe Asn Phe His Asn Asn
           195                 200                 205
   Gly Gln Met Ile Trp Gly Ala Thr Leu Asp Glu Ser Ala Ala Asn Pro
       210                 215                 220
   Ser Tyr Ser Glu Trp Ala Ala Lys Ala Thr Ala Thr Val Thr Gly Asp
   225                 230                 235                 240
   Cys Gly Gly Ala Thr Pro Thr Thr Thr Thr Thr Thr Thr Thr Ser Val
                   245                 250                 255
   Pro Thr Ala Thr Gly Ile Pro Val Pro Thr Gly Thr Tyr Asp Tyr Ile
               260                 265                 270
   Val Val Gly Ala Gly Ala Gly Gly Ile Pro Leu Ala Asp Lys Leu Ser
           275                 280                 285
   Glu Ala Gly Lys Ser Val Leu Leu Ile Glu Lys Gly Pro Pro Ser Ser
       290                 295                 300
   Gly Arg Trp Gly Gly Thr Leu Lys Pro Glu Trp Leu Lys Asp Thr Asn
   305                 310                 315                 320
   Leu Thr Arg Phe Asp Val Pro Gly Leu Cys Asn Glu Ile Trp Val Asn
                   325                 330                 335
   Ser Ala Gly Val Ala Cys Thr Asp Thr Asp Gln Met Ala Gly Cys Val
               340                 345                 350
   Leu Gly Gly Gly Thr Ala Val Asn Ala Gly Leu Trp Trp Lys Pro Tyr
           355                 360                 365
   Asn Leu Asp Trp Asp Tyr Asn Phe Pro Arg Gly Trp Lys Ser Arg Asp
       370                 375                 380
   Met Ala Ala Ala Thr Arg Arg Val Phe Ser Arg Ile Pro Gly Thr Asp
   385                 390                 395                 400
   Asn Pro Ser Met Asp Gly Lys Arg Tyr Leu Gln Gln Gly Phe Glu Ile
                   405                 410                 415
   Leu Ala Gly Gly Leu Lys Ala Ala Gly Trp Thr Glu Val Thr Ala Asn
               420                 425                 430
   Asp Ala Pro Asn Lys Lys Asn His Thr Tyr Ser His Ser Pro Phe Met
           435                 440                 445
   Phe Ser Gly Gly Glu Arg Gly Gly Pro Met Gly Thr Tyr Leu Val Ser
       450                 455                 460
   Ala Ser Arg Arg Lys Asn Phe His Leu Trp Thr Gly Thr Ala Val Lys
   465                 470                 475                 480
   Arg Val Val Arg Thr Gly Gly His Ile Thr Gly Leu Glu Val Glu Pro
                   485                 490                 495
   Phe Val Asn Gly Gly Tyr Thr Gly Val Val Asn Val Thr Ser Ile Thr
               500                 505                 510
   Gly Arg Val Val Leu Ser Ala Gly Ala Phe Gly Ser Ala Lys Ile Leu
           515                 520                 525
   Leu Arg Ser Gly Ile Gly Pro Glu Asp Gln Leu Glu Ile Val Lys Ser
       530                 535                 540
   Ser Thr Asp Gly Pro Thr Met Ile Ser Asp Ser Ser Trp Ile Thr Leu
   545                 550                 555                 560
   Pro Val Gly Tyr Asn Leu Glu Asp His Thr Asn Thr Asp Thr Val Val
   565 570 575
   Thr His Pro Asp Val Val Phe Tyr Asp Phe Tyr Glu Ala Gly His Pro
   580 585 590
   Asn Val Thr Asp Lys Asp Leu Tyr Leu Asn Ser Arg Ala Gly Ile Leu
   595 600 605
   Ala Gln Ala Ala Pro Asn Ile Gly Pro Met Phe Trp Glu Glu Ile Lys
   610 615 620
   Gly Lys Asp Gly Val Val Arg Gln Leu Gln Trp Thr Ala Arg Val Glu
   625 630 635 640
   Gly Ser Ala Gly Thr Pro Asn Gly Tyr Ala Met Thr Met Ser Gln Tyr
   645 650 655
   Leu Gly Arg Gly Ala Lys Ser Arg Gly Arg Met Thr Ile Thr Lys Ala
   660 665 670
   Leu Thr Thr Val Val Ser Thr Val Pro Tyr Leu Gln Asp Lys Asn Asp
   675 680 685
   Val Glu Ala Val Ile Gln Gly Ile Lys Asn Leu Gln Ala Ala Leu Ser
   690 695 700
   Asn Val Lys Asn Leu Thr Trp Thr Tyr Pro Pro Ser Asn Thr Thr Val
   705 710 715 720
   Glu Asp Phe Val Asn Asn Met Leu Val Ser Tyr Thr Asn Arg Arg Ser
   725 730 735
   Asn His Trp Ile Gly Thr Asn Lys Leu Gly Thr Asp Asp Gly Arg Ser
   740 745 750
   Arg Gly Gly Ser Ala Val Val Asp Leu Asn Thr Lys Val Tyr Gly Thr
   755 760 765
   Asp Asn Leu Phe Val Val Asp Ala Gly Ile Phe Pro Gly His Ile Thr
   770 775 780
   Thr Asn Pro Thr Ser Tyr Ile Val Ile Ala Ala Glu Arg Ala Ser Glu
   785 790 795 800
   Arg Ile Leu Asp Leu Pro Pro Ala Arg Ala Gln Pro Arg Phe Ala Gln
   805 810 815
   Cys Gly Gly Arg Thr Trp Thr Gly Ser Phe Gln Cys Ala Ala Pro Tyr
   820 825 830
   Thr Cys Gln Tyr Arg Asn Glu Arg Tyr Ser Gln Cys Arg
   835 840 845
<210> 3
   <211> 787
   <212> PRT
   <213> Corynascus thermophilus
<400> 3
   Met Lys Leu Leu Ser Arg Val Gly Ala Thr Ala Leu Ala Ala Thr Leu
   1 5 10 15
   Ser Leu Lys Gln Cys Ala Ala Gln Met Thr Glu Gly Thr Tyr Thr His
   20 25 30
   Glu Ala Thr Gly Ile Thr Phe Lys Thr Trp Thr Pro Ser Asp Gly Ser
   35 40 45
   Thr Phe Thr Phe Gly Leu Ala Leu Pro Gly Asp Ala Leu Thr Asn Asp
   50 55 60
   Ala Thr Glu Tyr Ile Gly Leu Leu Arg Cys Gln Ile Thr Asp Pro Ser
   65 70 75 80
   Ser Pro Gly Tyr Cys Gly Ile Ser His Gly Gln Ser Gly Gln Met Thr
   85 90 95
   Gln Ala Leu Leu Leu Val Ala Trp Ala Ser Glu Asp Val Val Tyr Thr
   100 105 110
   Ser Phe Arg Tyr Ala Thr Gly Tyr Thr Leu Pro Glu Leu Tyr Thr Gly
   115 120 125
   Asp Ala Lys Leu Thr Gln Ile Ala Ser Ser Val Ser Gly Asp Ser Phe
   130 135 140
   Glu Val Leu Phe Arg Cys Glu Asn Cys Phe Ser Trp Asp Gln Asn Gly
   145 150 155 160
   Ala Thr Gly Ser Val Ser Thr Ser Asn Gly Ala Leu Val Leu Gly Tyr
   165 170 175
   Ala Ala Ser Lys Ser Gly Leu Thr Gly Ala Thr Cys Pro Asp Thr Ala
   180 185 190
   Glu Phe Gly Phe His Asn Asn Gly Phe Gly Gln Trp Gly Ala Val Leu
           195                 200                 205
   Glu Gly Ala Thr Ser Asp Ser Tyr Glu Glu Trp Ala Gln Leu Ala Thr
       210                 215                 220
   Ile Thr Pro Pro Thr Thr Cys Asp Gly Asn Gly Pro Gly Asp Lys Val
   225                 230                 235                 240
   Cys Val Pro Ala Pro Glu Asp Thr Tyr Asp Tyr Ile Val Val Gly Ala
                   245                 250                 255
   Gly Ala Gly Gly Ile Thr Val Ala Asp Lys Leu Ser Glu Ala Gly His
               260                 265                 270
   Lys Val Leu Leu Ile Glu Lys Gly Pro Pro Ser Thr Gly Leu Trp Asn
           275                 280                 285
   Gly Thr Met Lys Pro Glu Trp Leu Glu Gly Thr Asp Leu Thr Arg Phe
       290                 295                 300
   Asp Val Pro Gly Leu Cys Asn Gln Ile Trp Val Asp Ser Ala Gly Ile
   305                 310                 315                 320
   Ala Cys Thr Asp Thr Asp Gln Met Ala Gly Cys Val Leu Gly Gly Gly
                   325                 330                 335
   Thr Ala Val Asn Ala Gly Leu Trp Trp Lys Pro His Pro Ala Asp Trp
               340                 345                 350
   Asp Asp Asn Phe Pro His Gly Trp Lys Ser Ser Asp Leu Ala Asp Ala
           355                 360                 365
   Thr Glu Arg Val Phe Ser Arg Ile Pro Gly Thr Trp His Pro Ser Gln
       370                 375                 380
   Asp Gly Lys Leu Tyr Arg Gln Glu Gly Phe Glu Val Ile Ser Gln Gly
   385                 390                 395                 400
   Leu Ala Asn Ala Gly Trp Arg Glu Val Asp Ala Asn Gln Glu Pro Ser
                   405                 410                 415
   Glu Lys Asn Arg Thr Tyr Ser His Ser Val Phe Met Phe Ser Gly Gly
               420                 425                 430
   Glu Arg Gly Gly Pro Leu Ala Thr Tyr Leu Ala Ser Ala Ala Gln Arg
           435                 440                 445
   Ser Asn Phe Asn Leu Trp Val Asn Thr Ser Val Arg Arg Ala Ile Arg
       450                 455                 460
   Thr Gly Pro Arg Val Ser Gly Val Glu Leu Glu Cys Leu Ala Asp Gly
   465                 470                 475                 480
   Gly Phe Asn Gly Thr Val Asn Leu Lys Glu Gly Gly Gly Val Ile Phe
                   485                 490                 495
   Ser Ala Gly Ala Phe Gly Ser Ala Lys Leu Leu Leu Arg Ser Gly Ile
               500                 505                 510
   Gly Pro Glu Asp Gln Leu Glu Ile Val Ala Ser Ser Lys Asp Gly Glu
           515                 520                 525
   Thr Phe Ile Ser Lys Asn Asp Trp Ile Lys Leu Pro Val Gly His Asn
       530                 535                 540
   Leu Ile Asp His Leu Asn Thr Asp Leu Ile Ile Thr His Pro Asp Val
   545                 550                 555                 560
   Val Phe Tyr Asp Phe Tyr Ala Ala Trp Asp Asn Pro Ile Thr Glu Asp
                   565                 570                 575
   Lys Glu Ala Tyr Leu Asn Ser Arg Ser Gly Ile Leu Ala Gln Ala Ala
               580                 585                 590
   Pro Asn Ile Gly Pro Leu Met Trp Glu Glu Val Thr Pro Ser Asp Gly
           595                 600                 605
   Ile Thr Arg Gln Phe Gln Trp Thr Cys Arg Val Glu Gly Asp Ser Ser
       610                 615                 620
   Lys Thr Asn Ser Thr His Ala Met Thr Leu Ser Gln Tyr Leu Gly Arg
   625                 630                 635                 640
   Gly Val Val Ser Arg Gly Arg Met Gly Ile Thr Ser Gly Leu Thr Thr
                   645                 650                 655
   Thr Val Ala Glu His Pro Tyr Leu His Asn Asp Gly Asp Leu Glu Ala
               660                 665                 670
   Val Ile Gln Gly Ile Gln Asn Val Val Asp Ala Leu Ser Gln Val Pro
           675                 680                 685
   Asp Leu Glu Trp Val Leu Pro Pro Pro Asn Thr Thr Val Glu Glu Tyr
       690                 695                 700
   Val Asn Ser Leu Ile Val Ser Pro Ala Asn Arg Arg Ala Asn His Trp
   705                 710                 715                 720
   Met Gly Thr Ala Lys Met Gly Leu Asp Asp Gly Arg Ser Gly Gly Ser
                   725                 730                 735
   Ala Val Val Asp Leu Asn Thr Lys Val Tyr Gly Thr Asp Asn Leu Phe
               740                 745                 750
   Val Val Asp Ala Ser Ile Phe Pro Gly Met Ser Thr Gly Asn Pro Ser
           755                 760                 765
   Ala Met Ile Val Ile Val Ala Glu Gln Ala Ala Gln Arg Ile Leu Ser
       770                 775                 780
   Leu Arg Tyr
   785
<210> 4
   <211> 829
   <212> PRT
   <213> Neurospora crassa
<400> 4
   Met Arg Thr Thr Ser Ala Phe Leu Ser Gly Leu Ala Ala Val Ala Ser
   1               5                   10                  15
   Leu Leu Ser Pro Ala Phe Ala Gln Thr Ala Pro Lys Thr Phe Thr His
               20                  25                  30
   Pro Asp Thr Gly Ile Val Phe Asn Thr Trp Ser Ala Ser Asp Ser Gln
           35                  40                  45
   Thr Lys Gly Gly Phe Thr Val Gly Met Ala Leu Pro Ser Asn Ala Leu
       50                  55                  60
   Thr Thr Asp Ala Thr Glu Phe Ile Gly Tyr Leu Glu Cys Ser Ser Ala
   65                  70                  75                  80
   Lys Asn Gly Ala Asn Ser Gly Trp Cys Gly Val Ser Leu Arg Gly Ala
                   85                  90                  95
   Met Thr Asn Asn Leu Leu Ile Thr Ala Trp Pro Ser Asp Gly Glu Val
               100                 105                 110
   Tyr Thr Asn Leu Met Phe Ala Thr Gly Tyr Ala Met Pro Lys Asn Tyr
           115                 120                 125
   Ala Gly Asp Ala Lys Ile Thr Gln Ile Ala Ser Ser Val Asn Ala Thr
       130                 135                 140
   His Phe Thr Leu Val Phe Arg Cys Gln Asn Cys Leu Ser Trp Asp Gln
   145                 150                 155                 160
   Asp Gly Val Thr Gly Gly Ile Ser Thr Ser Asn Lys Gly Ala Gln Leu
                   165                 170                 175
   Gly Trp Val Gln Ala Phe Pro Ser Pro Gly Asn Pro Thr Cys Pro Thr
               180                 185                 190
   Gln Ile Thr Leu Ser Gln His Asp Asn Gly Met Gly Gln Trp Gly Ala
           195                 200                 205
   Ala Phe Asp Ser Asn Ile Ala Asn Pro Ser Tyr Thr Ala Trp Ala Ala
       210                 215                 220
   Lys Ala Thr Lys Thr Val Thr Gly Thr Cys Ser Gly Pro Val Thr Thr
   225                 230                 235                 240
   Ser Ile Ala Ala Thr Pro Val Pro Thr Gly Val Ser Phe Asp Tyr Ile
                   245                 250                 255
   Val Val Gly Gly Gly Ala Gly Gly Ile Pro Val Ala Asp Lys Leu Ser
               260                 265                 270
   Glu Ser Gly Lys Ser Val Leu Leu Ile Glu Lys Gly Phe Ala Ser Thr
           275                 280                 285
   Gly Glu His Gly Gly Thr Leu Lys Pro Glu Trp Leu Asn Asn Thr Ser
       290                 295                 300
   Leu Thr Arg Phe Asp Val Pro Gly Leu Cys Asn Gln Ile Trp Lys Asp
   305                 310                 315                 320
   Ser Asp Gly Ile Ala Cys Ser Asp Thr Asp Gln Met Ala Gly Cys Val
                   325                 330                 335
   Leu Gly Gly Gly Thr Ala Ile Asn Ala Gly Leu Trp Tyr Lys Pro Tyr
               340                 345                 350
   Thr Lys Asp Trp Asp Tyr Leu Phe Pro Ser Gly Trp Lys Gly Ser Asp
           355                 360                 365
   Ile Ala Gly Ala Thr Ser Arg Ala Leu Ser Arg Ile Pro Gly Thr Thr
   370 375 380
   Thr Pro Ser Gln Asp Gly Lys Arg Tyr Leu Gln Gln Gly Phe Glu Val
   385 390 395 400
   Leu Ala Asn Gly Leu Lys Ala Ser Gly Trp Lys Glu Val Asp Ser Leu
   405 410 415
   Lys Asp Ser Glu Gln Lys Asn Arg Thr Phe Ser His Thr Ser Tyr Met
   420 425 430
   Tyr Ile Asn Gly Glu Arg Gly Gly Pro Leu Ala Thr Tyr Leu Val Ser
   435 440 445
   Ala Lys Lys Arg Ser Asn Phe Lys Leu Trp Leu Asn Thr Ala Val Lys
   450 455 460
   Arg Val Ile Arg Glu Gly Gly His Ile Thr Gly Val Glu Val Glu Ala
   465 470 475 480
   Phe Arg Asn Gly Gly Tyr Ser Gly Ile Ile Pro Val Thr Asn Thr Thr
   485 490 495
   Gly Arg Val Val Leu Ser Ala Gly Thr Phe Gly Ser Ala Lys Ile Leu
   500 505 510
   Leu Arg Ser Gly Ile Gly Pro Lys Asp Gln Leu Glu Val Val Lys Ala
   515 520 525
   Ser Ala Asp Gly Pro Thr Met Val Ser Asn Ser Ser Trp Ile Asp Leu
   530 535 540
   Pro Val Gly His Asn Leu Val Asp His Thr Asn Thr Asp Thr Val Ile
   545 550 555 560
   Gln His Asn Asn Val Thr Phe Tyr Asp Phe Tyr Lys Ala Trp Asp Asn
   565 570 575
   Pro Asn Thr Thr Asp Met Asn Leu Tyr Leu Asn Gly Arg Ser Gly Ile
   580 585 590
   Phe Ala Gln Ala Ala Pro Asn Ile Gly Pro Leu Phe Trp Glu Glu Ile
   595 600 605
   Thr Gly Ala Asp Gly Ile Val Arg Gln Leu His Trp Thr Ala Arg Val
   610 615 620
   Glu Gly Ser Phe Glu Thr Pro Asp Gly Tyr Ala Met Thr Met Ser Gln
   625 630 635 640
   Tyr Leu Gly Arg Gly Ala Thr Ser Arg Gly Arg Met Thr Leu Ser Pro
   645 650 655
   Thr Leu Asn Thr Val Val Ser Asp Leu Pro Tyr Leu Lys Asp Pro Asn
   660 665 670
   Asp Lys Ala Ala Val Val Gln Gly Ile Val Asn Leu Gln Lys Ala Leu
   675 680 685
   Ala Asn Val Lys Gly Leu Thr Trp Ala Tyr Pro Ser Ala Asn Gln Thr
   690 695 700
   Ala Ala Asp Phe Val Asp Lys Gln Pro Val Thr Tyr Gln Ser Arg Arg
   705 710 715 720
   Ser Asn His Trp Met Gly Thr Asn Lys Met Gly Thr Asp Asp Gly Arg
   725 730 735
   Ser Gly Gly Thr Ala Val Val Asp Thr Asn Thr Arg Val Tyr Gly Thr
   740 745 750
   Asp Asn Leu Tyr Val Val Asp Ala Ser Ile Phe Pro Gly Val Pro Thr
   755 760 765
   Thr Asn Pro Thr Ala Tyr Ile Val Val Ala Ala Glu His Ala Ala Ala
   770 775 780
   Lys Ile Leu Ala Gln Pro Ala Asn Glu Ala Val Pro Lys Trp Gly Trp
   785 790 795 800
   Cys Gly Gly Pro Thr Tyr Thr Gly Ser Gln Thr Cys Gln Ala Pro Tyr
   805 810 815
   Lys Cys Glu Lys Gln Asn Asp Trp Tyr Trp Gln Cys Val
   820 825
<210> 5
   <211> 828
   <212> PRT
   <213> Myriococcum thermophilum
<400> 5
<210> 6
   <211> 829
   <212> PRT
   <213> Stachybotrys bisbyi
<400> 6
   Met Leu Phe Lys Leu Ser Asn Trp Leu Leu Ala Leu Ala Leu Phe Val
   1               5                   10                  15
   Gly Asn Val Val Ala Gln Leu Val Gly Pro Thr Pro Tyr Thr Asp Pro
               20                  25                  30
   Asp Thr Gly Ile Val Phe Gln Ser Trp Val Asn Pro Ala Gly Thr Leu
           35                  40                  45
   Lys Phe Gly Tyr Thr Tyr Pro Ala Asn Ala Ala Thr Val Ala Ala Thr
       50                  55                  60
   Glu Phe Ile Gly Phe Leu Glu Cys Gln Gly Ala Gly Trp Cys Ser Val
   65                  70                  75                  80
   Ser Leu Gly Gly Ser Met Leu Asn Lys Pro Leu Val Val Ala Tyr Pro
                   85                  90                  95
   Ser Gly Asp Glu Val Leu Ala Ser Leu Lys Trp Ala Thr Gly Tyr Ala
               100                 105                 110
   Asn Pro Glu Pro Tyr Gly Gly Asn His Lys Leu Ser Gln Ile Ser Ser
           115                 120                 125
   Ser Val Thr Ser Ala Gly Phe Arg Val Val Tyr Arg Cys Glu Gly Cys
       130                 135                 140
   Leu Ala Trp Asn Tyr Gln Gly Ile Glu Gly Gly Ser Pro Thr Asn Gly
   145                 150                 155                 160
   Ala Ser Met Pro Ile Gly Trp Ala Tyr Ser Ala Ser Ser Val Leu Asn
                   165                 170                 175
   Gly Asp Cys Val Asp Asn Thr Val Leu Ile Gln His Asp Thr Phe Gly
               180                 185                 190
   Asn Tyr Gly Phe Val Pro Asp Glu Ser Ser Leu Arg Thr Glu Tyr Asn
           195                 200                 205
   Asp Trp Thr Glu Leu Pro Thr Arg Val Val Arg Gly Asp Cys Gly Gly
       210                 215                 220
   Ser Thr Thr Thr Ser Ser Val Pro Ser Ser Thr Ala Pro Pro Gln Gly
   225                 230                 235                 240
   Thr Gly Ile Pro Val Pro Thr Gly Ala Ser Tyr Asp Tyr Ile Val Val
                   245                 250                 255
   Gly Ser Gly Ala Gly Gly Ile Pro Ile Ala Asp Lys Leu Thr Glu Ala
               260                 265                 270
   Gly Lys Lys Val Leu Leu Ile Glu Lys Gly Pro Pro Ser Ser Gly Arg
           275                 280                 285
   Tyr Asp Gly Lys Leu Lys Pro Thr Trp Leu Glu Gly Thr Asn Leu Thr
       290                 295                 300
   Arg Phe Asp Val Pro Gly Leu Cys Asn Gln Ile Trp Val Asp Ser Ala
   305                 310                 315                 320
   Gly Ile Ala Cys Arg Asp Thr Asp Gln Met Ala Gly Cys Val Leu Gly
                   325                 330                 335
   Gly Gly Thr Ala Val Asn Ala Gly Leu Trp Trp Lys Pro Asn Pro Ile
               340                 345                 350
   Asp Trp Asp Tyr Asn Phe Pro Ser Gly Trp Lys Ser Ser Glu Met Ile
           355                 360                 365
   Gly Ala Thr Asn Arg Val Phe Ser Arg Ile Gly Gly Thr Thr Val Pro
       370                 375                 380
   Ser Gln Asp Gly Lys Thr Tyr Tyr Gln Gln Gly Phe Asn Val Leu Ser
   385                 390                 395                 400
   Ser Gly Leu Lys Ala Ala Gly Trp Thr Ser Val Ser Leu Asn Asn Ala
                   405                 410                 415
   Pro Ala Gln Lys Asn Arg Thr Tyr Gly Ala Gly Pro Phe Met Phe Ser
               420                 425                 430
   Gly Gly Glu Arg Gly Gly Pro Leu Ala Thr Tyr Leu Ala Thr Ala Lys
           435                 440                 445
   Lys Arg Gly Asn Phe Asp Leu Trp Thr Asn Thr Gln Val Lys Arg Val
       450                 455                 460
   Ile Arg Gln Gly Gly His Val Thr Gly Val Glu Val Glu Asn Tyr Asn
   465                 470                 475                 480
   Gly Asp Gly Tyr Lys Gly Thr Val Lys Val Thr Pro Val Ser Gly Arg
                   485                 490                 495
   Val Val Leu Ser Ala Gly Thr Phe Gly Ser Ala Lys Leu Leu Leu Arg
               500                 505                 510
   Ser Gly Ile Gly Pro Lys Asp Gln Leu Ala Ile Val Lys Asn Ser Thr
           515                 520                 525
   Asp Gly Pro Thr Met Ala Ser Glu Arg Asp Trp Ile Asn Leu Pro Val
       530                 535                 540
   Gly Tyr Asn Leu Glu Asp His Thr Asn Thr Asp Ile Val Ile Ser His
   545                 550                 555                 560
   Pro Asp Val Val His Tyr Asp Phe Tyr Glu Ala Trp Thr Ala Pro Ile
                   565                 570                 575
   Glu Ser Asp Lys Thr Ala Tyr Leu Gly Lys Arg Ser Gly Ile Leu Ala
               580                 585                 590
   Gln Ala Ala Pro Asn Ile Gly Pro Leu Phe Phe Asp Glu Val Arg Gly
           595                 600                 605
   Ala Asp Asn Ile Val Arg Ser Ile Gln Tyr Thr Ala Arg Val Glu Gly
       610                 615                 620
   Asn Ser Val Val Pro Asn Gly Lys Ala Met Val Ile Ser Gln Tyr Leu
   625                 630                 635                 640
   Gly Arg Gly Ala Val Ser Arg Gly Arg Met Thr Ile Ser Gln Gly Leu
                   645                 650                 655
   Asn Thr Ile Val Ser Thr Ala Pro Tyr Leu Ser Asn Val Asn Asp Leu
               660                 665                 670
   Glu Ala Val Ile Lys Ser Leu Glu Asn Ile Ala Asn Ser Leu Thr Ser
   675 680 685
   Lys Val Lys Asn Leu Lys Ile Glu Trp Pro Ala Ser Gly Thr Ser Ile
   690 695 700
   Arg Asp His Val Thr Asn Met Pro Leu Asp Pro Ala Thr Arg Arg Ala
   705 710 715 720
   Asn His Trp Ile Gly Thr Asn Lys Ile Gly Thr Lys Asp Gly Arg Leu
   725 730 735
   Thr Gly Gly Asp Ser Val Val Asp Leu Asn Thr Lys Val Tyr Gly Thr
   740 745 750
   Asp Asn Leu Phe Val Val Asp Ala Ser Ile Phe Pro Gly Met Val Thr
   755 760 765
   Thr Asn Pro Ser Ala Tyr Ile Val Ile Ala Ala Glu His Ala Ala Ser
   770 775 780
   Lys Ile Leu Ser Leu Pro Thr Ala Lys Ala Ala Ala Lys Tyr Glu Gln
   785 790 795 800
   Cys Gly Gly Leu Glu Tyr Asn Gly Asn Phe Gln Cys Ala Ser Gly Leu
   805 810 815
   Thr Cys Thr Trp Leu Asn Asp Tyr Tyr Trp Gln Cys Thr
   820 825
<210> 7
   <211> 771
   <212> PRT
   <213> Athelia rolfsii
<400> 7
   Met Leu Ser Arg Leu Val Leu Asn Leu Leu Ala Ile Thr Val Ile Gly
   1 5 10 15
   Val Phe Gly Gln Ser Ser Ser Ser Tyr Thr Asp Asn Gly Ile Asn Phe
   20 25 30
   Gln Gly Ile Thr Asp Pro Thr Tyr Gly Val Thr Tyr Gly Ala Val Phe
   35 40 45
   Pro Pro Ala Ser Val Asp Ser Asp Glu Phe Ile Gly Glu Ile Ala Ala
   50 55 60
   Pro Val Ala Ala Lys Trp Ile Gly Leu Ser Leu Gly Gly Ala Met Ile
   65                  70                  75                  80
   Asn Asn Leu Leu Ile Val Ala Trp Pro Asn Asn Asn Glu Ile Val Phe
                   85                  90                  95
   Ser Ser Arg Tyr Thr Thr Gly Tyr Val Leu Pro Thr Ile Tyr Ser Gly
               100                 105                 110
   Pro Lys Ile Thr Thr Ile Ser Ser Ser Val Asn Ser Thr His Trp Lys
           115                 120                 125
   Trp Ile Tyr Arg Cys Gln Asn Cys Thr Thr Trp Ser Gly Gly Ser Leu
       130                 135                 140
   Ala Ala Asn Gly Ser Ala Val Trp Ala Trp Ala Tyr Ser Ser Ala Ala
   145                 150                 155                 160
   Val Asp Thr Pro Ser Ser Pro Ser Ser Ser Phe Asp Glu His Thr Asp
                   165                 170                 175
   Phe Gly Phe Phe Gly Glu Ile Thr Ser Asn Ala His Val Ser Gln Ser
               180                 185                 190
   Val Tyr Glu Gln Tyr Leu Thr Gly Thr Gly Val Thr Ser Thr Ser Ser
           195                 200                 205
   Ser Thr Ser Thr Thr Thr Ser Thr Ser Thr Thr Thr Ser Thr Ser Ser
       210                 215                 220
   Ala Pro Thr Val Ser Ala Thr Pro Tyr Asp Tyr Ile Ile Val Gly Ala
   225                 230                 235                 240
   Gly Pro Ala Gly Ile Ile Ala Ala Asp Arg Leu Ser Glu Ala Gly Lys
                   245                 250                 255
   Lys Val Leu Leu Leu Glu Arg Gly Gly Pro Ser Thr Lys Glu Thr Gly
               260                 265                 270
   Gly Thr Tyr Thr Ala Pro Trp Ala Ala Ser Ser Gly Leu Thr Lys Phe
           275                 280                 285
   Asp Ile Pro Gly Val Phe Glu Ser Leu Phe Thr Asp Ser Asn Ser Phe
       290                 295                 300
   Trp Trp Cys Lys Asp Ile Thr Val Phe Ala Gly Cys Leu Thr Gly Gly
   305                 310                 315                 320
   Gly Thr Ala Ile Asn Gly Ala Leu Tyr Trp Tyr Pro Thr Asp Leu Asp
                   325                 330                 335
   Phe Ser Thr Ala Asn Gly Trp Pro Ser Ser Trp Thr Ala His Gln Lys
               340                 345                 350
   Tyr Thr Asp Leu Val Ser Ser Arg Leu Pro Ser Ser Asp His Pro Ser
           355                 360                 365
   Thr Asp Gly Lys Arg Tyr Leu Glu Gln Ser Ala Ala Val Val Ala Gln
       370                 375                 380
   Leu Leu Asn Gly Gln Gly Tyr Arg Asn Lys Thr Ile Asn Asn Ala Pro
   385                 390                 395                 400
   Asn Ala Lys Asp His Val Tyr Gly Tyr Ser Ala Phe Asp Phe Leu Asn
                   405                 410                 415
   Gly Lys Arg Ala Gly Pro Val Ala Thr Tyr Leu Gln Thr Ala Lys Thr
               420                 425                 430
   Arg Asn Asn Phe His Phe Lys Asn Tyr Val Leu Val Ser Asn Val Val
           435                 440                 445
   Arg Asn Gly Ser Thr Ile Thr Gly Val Lys Thr Asn Asp Thr Ser Leu
       450                 455                 460
   Gly Pro Asn Gly Val Ile Pro Leu Thr Lys Asn Gly Arg Val Ile Leu
   465                 470                 475                 480
   Ser Ala Gly Ser Leu Ser Ser Pro Arg Ile Leu Phe Gln Ser Gly Ile
                   485                 490                 495
   Gly Pro Thr Asp Met Leu Thr Leu Val Gln Asn Asn Pro Thr Ala Ser
               500                 505                 510
   Ala Asn Leu Pro Ser Gln Ser Gln Trp Ile His Leu Pro Val Gly Tyr
           515                 520                 525
   Asn Val Ala Asp Ala Pro Ser Ile Asn Phe Val Phe Thr His Pro Ser
       530                 535                 540
   Ile Asp Ala Tyr Asp Asn Trp Ala Asn Val Trp Thr Asn Pro Arg Ser
   545                 550                 555                 560
   Thr Asp Ala Glu Gln Tyr Leu Lys Asn Gln Ser Gly Val Leu Ala Ala
   565 570 575
   Ser Ser Pro Lys Leu Asn Phe Trp Arg Ala Tyr Gly Gly Ser Asp Gly
   580 585 590
   Arg Thr Arg Trp Met Gln Gly Thr Val Arg Pro Gly Ala Ala Ser Ile
   595 600 605
   Asn Thr Thr Tyr Asn Tyr Asn Ala Ser Gln Ile Phe Thr Ile Thr Thr
   610 615 620
   Tyr Val Ser Thr Gly Ile Thr Ser Arg Gly Arg Ile Gly Val Thr Ser
   625 630 635 640
   Ser Leu Asn Val Leu Pro Leu Val Asn Pro Trp Leu Val Asp Pro Val
   645 650 655
   Asp Glu Lys Val Leu Thr Gln Ser Leu Glu Asp Leu Val Ser Asn Met
   660 665 670
   Lys Ser Val Pro Gly Leu Thr Met Ile Met Pro Asp Asn Thr Thr Ser
   675 680 685
   Ile Ala Asp Tyr Val Lys Asn Tyr Asp Arg Ala Ser Met Asn Ser Asn
   690 695 700
   His Trp Val Gly Ser Asn Lys Ile Ala Ser Asn Ala Thr Glu Gly Val
   705 710 715 720
   Val Asp Glu His Thr Lys Val Phe Arg Thr Asp Asn Leu Phe Ile Val
   725 730 735
   Asp Ala Ser Ile Ile Pro Ser Leu Pro Met Gly Asn Pro Gln Gly Ala
   740 745 750
   Leu Met Ser Ala Ala Glu Gln Ala Val Ala Lys Ile Leu Ala Leu Ala
   755 760 765
   Gly Gly Pro
   770
<210> 8
   <211> 774
   <212> PRT
   <213> Gelatoporia subvermispora
<400> 8
   Met Phe Gly Arg Phe Leu Leu Ala Leu Leu Pro Leu Val Gly Ser Val
   1               5                   10                  15
   Leu Ser Gln Ser Gly Ser Ser Tyr Thr Asp Pro Asp Asn Gly Phe Val
               20                  25                  30
   Phe Asn Gly Ile Thr Asp Pro Val Tyr Gly Val Thr Tyr Gly Val Val
           35                  40                  45
   Phe Pro Glu Pro Ser Ser Ser Gly Thr Tyr Pro Asp Glu Phe Ile Gly
       50                  55                  60
   Glu Ile Val Ala Pro Leu Thr Ala Glu Trp Ile Gly Val Ser Phe Gly
   65                  70                  75                      80
   Gly Ala Met Leu Asp Cys Leu Leu Leu Val Ala Trp Pro Asn Gln Asn
                   85                  90                  95
   Ser Ile Val Ala Ser Thr Arg Tyr Ala Thr Asp Tyr Val Gln Pro Thr
               100                 105                 110
   Glu Tyr Asp Gly Pro Val Leu Thr Thr Leu Pro Ser Ser Tyr Val Asn
           115                 120                 125
   Ser Thr His Trp Lys Tyr Val Tyr Arg Cys Gln Asn Cys Thr Thr Trp
       130                 135                 140
   Gln Gly Gly Gly Ile Ser Leu Gly Gly Thr Gly Val Leu Ala Trp Ala
   145                 150                 155                     160
   Tyr Ser Asn Val Gly Val Asp Asp Pro Ser Asp Pro Glu Ser Asn Phe
                   165                 170                 175
   Leu Glu His Thr Asp Phe Gly Phe Phe Gly Glu Asn Phe Gly Gln Thr
               180                 185                 190
   Glu Asn Ala Asn Tyr Asn Asn Tyr Val Asn Gly Asn Pro Gly Thr Pro
           195                 200                 205
   Thr Ser Thr Pro Pro Thr Thr Ser Pro Thr Gly Pro Thr Thr Thr Ser
       210                 215                 220
   Pro Ala Ser Pro Pro Thr Ala Ser Ala Thr Pro Tyr Asp Tyr Ile Ile
   225                 230                 235                     240
   Val Gly Ala Gly Ala Gly Gly Ile Ile Ala Ala Asp Arg Leu Ser Gln
                   245                 250                 255
   Asn Asn Lys Lys Val Leu Leu Leu Glu Arg Gly Gly Pro Ser Thr Gly
               260                 265                 270
   Glu Thr Gly Gly Thr Tyr Val Ala Asp Trp Ala Glu Gly Thr Asn Leu
           275                 280                 285
   Thr Lys Phe Asp Ile Pro Gly Leu Phe Glu Ser Met Phe Asp Asp Pro
       290                 295                 300
   Asp Pro Trp Tyr Trp Cys Ser Asp Val Thr Phe Tyr Ala Gly Cys Leu
   305                 310                 315                 320
   Leu Gly Gly Gly Thr Ser Val Asn Gly Ala Leu Tyr Trp Tyr Pro Thr
                   325                 330                 335
   Asp Thr Asp Phe Ser Thr Ala Arg Gly Trp Pro Ser Ser Trp Ser Asn
               340                 345                 350
   His Gln Ala Tyr Thr Asn Ala Met Thr Gln Arg Leu Pro Ser Thr Asp
           355                 360                 365
   His Pro Ser Thr Asp Gly Glu Arg Tyr Leu Glu Gln Ser Ala Gln Val
       370                 375                 380
   Ala Met Gln Leu Leu Asn Ala Gln Gly Tyr Tyr Gln Ala Thr Ile Asn
   385                 390                 395                 400
   Asp Ser Pro Asp Ser Lys Asp His Val Tyr Gly Tyr Ser Ala Phe Asp
                   405                 410                 415
   Phe Ile Asn Gly Lys Arg Gly Gly Val Val Ala Thr Tyr Leu Gln Thr
               420                 425                 430
   Ala Asn Gln Arg Ser Asn Phe Val Tyr Lys Asp Tyr Thr Leu Val Ser
           435                 440                 445
   Ser Val Val Arg Asn Gly Ser Gln Ile Leu Gly Val Gln Thr Asn Asn
       450                 455                 460
   Thr Ala Ile Gly Pro Asn Gly Phe Ile Pro Leu Asn Pro Asn Gly Arg
   465                 470                 475                 480
   Val Ile Leu Ser Ala Gly Ser Phe Gly Thr Pro Arg Ile Leu Phe Gln
                   485                 490                 495
   Ser Gly Ile Gly Pro Thr Asp Met Leu Gln Thr Val Gln Gln Asn Ala
               500                 505                 510
   Ala Val Ala Ala Asn Met Pro Ser Glu Ser Asp Trp Ile Asn Leu Pro
           515                 520                 525
   Val Gly Met Asn Val Ser Asp Asn Pro Ser Ile Asn Leu Val Phe Thr
       530                 535                 540
   His Pro Ser Ile Asp Ala Tyr Asp Asn Trp Ala Asp Val Trp Thr Asp
   545                 550                 555                 560
   Pro Arg Pro Ala Asp Ala Ala Gln Tyr Leu Ala Ser Gln Ser Gly Val
                   565                 570                 575
   Phe Ala Gly Ala Ser Pro Lys Leu Asn Phe Trp Arg Asp Tyr Glu Gly
               580                 585                 590
   Ser Asp Gly Ile Gln Arg Ser Ala Gln Gly Thr Val Arg Pro Gly Ala
           595                 600                 605
   Ala Ser Val Asn Thr Thr Leu Pro Tyr Asn Ala Ser Gln Ile Phe Thr
       610                 615                 620
   Ile Thr Val Tyr Leu Ser Ser Gly Ile Thr Ser Arg Gly Arg Ile Gly
   625                 630                 635                 640
   Val Thr Ala Gly Leu Asn Ala Val Ala Leu Glu Asn Pro Trp Leu Thr
                   645                 650                 655
   Asp Pro Val Asp Lys Val Val Leu Ile Gln Ala Leu Glu Asp Val Ile
               660                 665                 670
   Ser Thr Leu Pro Ser Val Pro Asp Leu Thr Met Ile Thr Pro Asp Ser
           675                 680                 685
   Gly Met Thr Leu Glu Glu Tyr Val Asp Leu Tyr Asp Pro Ser Thr Met
       690                 695                 700
   Cys Ser Asn His Trp Val Gly Ser Ala Lys Met Gly Thr Ser Ser Asp
   705                 710                 715                 720
   Thr Ala Val Val Asp Glu Asn Ala Lys Val Phe Asn Thr Asp Asn Leu
                   725                 730                 735
   Phe Val Ile Asp Ala Ser Ile Val Pro Ser Leu Pro Val Gly Asn Pro
               740                 745                 750
   His Gly Thr Val Met Ser Ala Ala Glu Gln Ala Val Ala Asn Ile Leu
           755                 760                 765
   Ala Leu Ser Gly Gly Pro
   770
<210> 9
   <211> 773
   <212> PRT
   <213> Phanerochaete chrysosporium
<400> 9
   Met Leu Gly Arg Ser Leu Leu Ala Leu Leu Pro Phe Val Gly Leu Ala
   1 5 10 15
   Phe Ser Gln Ser Ala Ser Gln Phe Thr Asp Pro Thr Thr Gly Phe Gln
   20 25 30
   Phe Thr Gly Ile Thr Asp Pro Val His Asp Val Thr Tyr Gly Phe Val
   35 40 45
   Phe Pro Pro Leu Ala Thr Ser Gly Ala Gln Ser Thr Glu Phe Ile Gly
   50 55 60
   Glu Val Val Ala Pro Ile Ala Ser Lys Trp Ile Gly Ile Ala Leu Gly
   65 70 75 80
   Gly Ala Met Asn Asn Asp Leu Leu Leu Val Ala Trp Ala Asn Gly Asn
   85 90 95
   Gln Ile Val Ser Ser Thr Arg Trp Ala Thr Gly Tyr Val Gln Pro Thr
   100 105 110
   Ala Tyr Thr Gly Thr Ala Thr Leu Thr Thr Leu Pro Glu Thr Thr Ile
   115 120 125
   Asn Ser Thr His Trp Lys Trp Val Phe Arg Cys Gln Gly Cys Thr Glu
   130 135 140
   Trp Asn Asn Gly Gly Gly Ile Asp Val Thr Ser Gln Gly Val Leu Ala
   145 150 155 160
   Trp Ala Phe Ser Asn Val Ala Val Asp Asp Pro Ser Asp Pro Gln Ser
   165 170 175
   Thr Phe Ser Glu His Thr Asp Phe Gly Phe Phe Gly Ile Asp Tyr Ser
   180 185 190
   Thr Ala His Ser Ala Asn Tyr Gln Asn Tyr Leu Asn Gly Asp Ser Gly
   195 200 205
   Asn Pro Thr Thr Thr Ser Thr Lys Pro Thr Ser Thr Ser Ser Ser Val
   210 215 220
   Thr Thr Gly Pro Thr Val Ser Ala Thr Pro Tyr Asp Tyr Ile Ile Val
   225 230 235 240
   Gly Ala Gly Pro Gly Gly Ile Ile Ala Ala Asp Arg Leu Ser Glu Ala
   245 250 255
   Gly Lys Lys Val Leu Leu Leu Glu Arg Gly Gly Pro Ser Thr Lys Gln
   260 265 270
   Thr Gly Gly Thr Tyr Val Ala Pro Trp Ala Thr Ser Ser Gly Leu Thr
   275 280 285
   Lys Phe Asp Ile Pro Gly Leu Phe Glu Ser Leu Phe Thr Asp Ser Asn
   290 295 300
   Pro Phe Trp Trp Cys Lys Asp Ile Thr Val Phe Ala Gly Cys Leu Val
   305 310 315 320
   Gly Gly Gly Thr Ser Val Asn Gly Ala Leu Tyr Trp Tyr Pro Asn Asp
   325 330 335
   Gly Asp Phe Ser Ser Ser Val Gly Trp Pro Ser Ser Trp Thr Asn His
   340 345 350
   Ala Pro Tyr Thr Ser Lys Leu Ser Ser Arg Leu Pro Ser Thr Asp His
   355 360 365
   Pro Ser Thr Asp Gly Gln Arg Tyr Leu Glu Gln Ser Phe Asn Val Val
   370 375 380
   Ser Gln Leu Leu Lys Gly Gln Gly Tyr Asn Gln Ala Thr Ile Asn Asp
   385 390 395 400
   Asn Pro Asn Tyr Lys Asp His Val Phe Gly Tyr Ser Ala Phe Asp Phe
   405 410 415
   Leu Asn Gly Lys Arg Ala Gly Pro Val Ala Thr Tyr Leu Gln Thr Ala
   420 425 430
   Leu Ala Arg Pro Asn Phe Thr Phe Lys Thr Asn Val Met Val Ser Asn
   435 440 445
   Val Val Arg Asn Gly Ser Gln Ile Leu Gly Val Gln Thr Asn Asp Pro
   450 455 460
   Thr Leu Gly Pro Asn Gly Phe Ile Pro Val Thr Pro Lys Gly Arg Val
   465 470 475 480
   Ile Leu Ser Ala Gly Ala Phe Gly Thr Ser Arg Ile Leu Phe Gln Ser
   485 490 495
   Gly Ile Gly Pro Thr Asp Met Ile Gln Thr Val Gln Ser Asn Pro Thr
   500 505 510
   Ala Ala Ala Ala Leu Pro Pro Gln Asn Gln Trp Ile Asn Leu Pro Val
   515 520 525
   Gly Met Asn Ala Gln Asp Asn Pro Ser Ile Asn Leu Val Phe Thr His
   530 535 540
   Pro Ser Ile Asp Ala Tyr Glu Asn Trp Ala Asp Val Trp Ser Asn Pro
   545 550 555 560
   Arg Pro Ala Asp Ala Ala Gln Tyr Leu Ala Asn Gln Ser Gly Val Phe
   565 570 575
   Ala Gly Ala Ser Pro Lys Leu Asn Phe Trp Arg Ala Tyr Ser Gly Ser
   580 585 590
   Asp Gly Phe Thr Arg Tyr Ala Gln Gly Thr Val Arg Pro Gly Ala Ala
   595 600 605
   Ser Val Asn Ser Ser Leu Pro Tyr Asn Ala Ser Gln Ile Phe Thr Ile
   610 615 620
   Thr Val Tyr Leu Ser Thr Gly Ile Gln Ser Arg Gly Arg Ile Gly Ile
   625 630 635 640
   Asp Ala Ala Leu Arg Gly Thr Val Leu Thr Pro Pro Trp Leu Val Asn
   645 650 655
   Pro Val Asp Lys Thr Val Leu Leu Gln Ala Leu His Asp Val Val Ser
   660 665 670
   Asn Ile Gly Ser Ile Pro Gly Leu Thr Met Ile Thr Pro Asp Val Thr
   675 680 685
   Gln Thr Leu Glu Glu Tyr Val Asp Ala Tyr Asp Pro Ala Thr Met Asn
   690 695 700
   Ser Asn His Trp Val Ser Ser Thr Thr Ile Gly Ser Ser Pro Gln Ser
   705 710 715 720
   Ala Val Val Asp Ser Asn Val Lys Val Phe Gly Thr Asn Asn Leu Phe
   725 730 735
   Ile Val Asp Ala Gly Ile Ile Pro His Leu Pro Thr Gly Asn Pro Gln
   740 745 750
   Gly Thr Leu Met Ser Ala Ala Glu Gln Ala Ala Ala Lys Ile Leu Ala
   755 760 765
   Leu Ala Gly Gly Pro
   770
<210> 10
   <211> 768
   <212> PRT
   <213> Trametes versicolor
<400> 10
   Met Lys Phe Lys Ser Leu Leu Leu Ser Leu Leu Pro Leu Val Gly Ser
   1 5 10 15
   Val Tyr Ser Gln Val Ala Ala Pro Tyr Val Asp Ser Gly Asn Gly Phe
   20 25 30
   Val Phe Asp Gly Val Thr Asp Pro Val His Ser Val Thr Tyr Gly Ile
   35 40 45
   Val Leu Pro Gln Ala Ser Thr Ser Thr Glu Phe Ile Gly Glu Phe Val
   50 55 60
   Ala Pro Asn Glu Ala Gln Trp Ile Gly Leu Ala Leu Gly Gly Ala Met
   65 70 75 80
   Ile Gly Asn Leu Leu Leu Val Ala Trp Pro Asp Gly Asn Lys Ile Val
   85 90 95
   Ser Ser Pro Arg Tyr Ala Thr Gly Tyr Thr Leu Pro Ala Ala Tyr Ala
   100 105 110
   Gly Pro Thr Ile Thr Gln Leu Pro Ser Ser Ser Val Asn Ser Thr His
   115 120 125
   Trp Lys Phe Val Phe Arg Cys Gln Asn Cys Thr Ala Trp Asn Gly Gly
   130 135 140
   Ser Ile Asp Pro Ser Gly Thr Gly Val Phe Ala Trp Ala Phe Ser Asn
   145                 150                 155                 160
   Val Ala Val Asp Asp Pro Ser Asp Pro Asn Ser Ser Phe Ala Glu His
                   165                 170                 175
   Thr Asp Phe Gly Phe Phe Gly Ile Asn Phe Pro Asp Ala Gln Ser Ser
               180                 185                 190
   Asn Tyr Gln Asn Tyr Leu Ala Gly Asn Ala Gly Thr Pro Pro Pro Thr
           195                 200                 205
   Ser Val Pro Ser Gly Pro Ser Ser Thr Thr Thr Thr Thr Gly Pro Thr
       210                 215                 220
   Ala Thr Ala Thr Pro Phe Asp Tyr Ile Val Val Gly Ala Gly Pro Gly
   225                 230                 235                 240
   Gly Leu Val Thr Ala Asp Arg Leu Ser Glu Ala Gly Lys Lys Val Leu
                   245                 250                 255
   Leu Leu Glu Arg Gly Gly Pro Ser Thr Ala Glu Thr Gly Gly Thr Tyr
               260                 265                 270
   Asp Ala Thr Trp Ala Lys Ser Ala Asn Leu Thr Lys Phe Asp Val Pro
           275                 280                 285
   Gly Leu Phe Glu Thr Leu Phe Thr Asp Thr Asn Pro Phe Trp Trp Cys
       290                 295                 300
   Lys Asp Thr Asn Phe Phe Ala Gly Cys Leu Leu Gly Gly Gly Thr Ser
   305                 310                 315                 320
   Val Asn Gly Ala Leu Tyr Trp Tyr Pro Asn Ser Arg Asp Phe Ser Thr
                   325                 330                 335
   Ala Ser Gly Trp Pro Ser Ser Trp Gly Asn His Gln Pro Phe Thr Asp
               340                 345                 350
   Lys Leu Lys Gln Arg Leu Pro Ser Thr Asp His Pro Ser Ala Asp Gly
           355                 360                 365
   Gln Arg Tyr Leu Glu Gln Ser Ala Thr Val Val Gln Gln Leu Leu Ser
       370                 375                 380
   Gly Gln Gly Tyr Ser Gln Ile Thr Ile Asn Asp Asn Pro Asp Ser Lys
   385                 390                 395                 400
   Asp His Val Phe Gly Phe Ser Ala Phe Asp Phe Leu Asn Gly Gln Arg
   405 410 415
   Ala Gly Pro Val Ala Thr Tyr Phe Glu Thr Ala Leu Ala Arg Lys Asn
   420 425 430
   Phe Val Tyr Lys Asp Asn Val Leu Val Thr Gln Val Ile Arg Asn Gly
   435 440 445
   Ser Thr Ile Leu Gly Val Arg Thr Asn Asp Asn Thr Leu Gly Pro Asp
   450 455 460
   Gly Ile Val Pro Leu Asn Pro Asn Gly Arg Val Ile Leu Ser Gly Gly
   465 470 475 480
   Ser Phe Gly Thr Pro Arg Ile Leu Phe Gln Ser Gly Ile Gly Pro Thr
   485 490 495
   Asp Met Leu Gln Thr Val Gln Ser Asn Ala Gln Ala Ala Ala Asn Leu
   500 505 510
   Pro Pro Gln Ser Glu Trp Ile Asn Leu Pro Val Gly Gln Ser Val Ser
   515 520 525
   Asp Asn Pro Ser Ile Asn Leu Val Phe Thr His Pro Ser Ile Asp Ala
   530 535 540
   Tyr Asp Asn Trp Ala Asp Val Trp Ser Asn Pro Arg Pro Ala Asp Ala
   545 550 555 560
   Gln Gln Tyr Leu Gln Ser Arg Ser Gly Val Leu Ala Gly Ala Ser Pro
   565 570 575
   Lys Leu Asn Phe Trp Arg Ala Tyr Gly Gly Ser Asp Gly Ile Thr Arg
   580 585 590
   Tyr Ala Gln Gly Thr Val Arg Pro Gly Ala Ala Ser Val Asn Thr Ser
   595 600 605
   Val Ala Tyr Asn Ala Ser Glu Ile Phe Thr Ile Thr Leu Tyr Leu Ser
   610 615 620
   Asn Gly Ile Gln Ser Arg Gly Arg Ile Gly Val Asp Ala Ala Leu Asn
   625 630 635 640
   Ala Lys Ala Leu Val Asn Pro Trp Leu Thr Asn Ser Val Asp Lys Thr
   645 650 655
   Val Leu Leu Gln Ala Leu His Asp Val Thr Ser Thr Met Lys Asn Val
                   660                 665                 670
   Pro Gly Leu Thr Met Ile Thr Pro Asp Asn Thr Met Thr Leu Glu Gln
           675                 680                 685
   Tyr Val Ala Ala Tyr Asp Pro Ala Thr Met Cys Ser Asn His Trp Val
       690                 695                 700
   Gly Ala Ala Lys Met Gly Thr Ser Ser Ser Thr Ala Val Val Asp Glu
   705                 710                 715                 720
   Asn Ala Lys Val Phe Asn Thr Asp Asn Leu Phe Ile Val Asp Ala Ser
                   725                 730                 735
   Ile Ile Pro Ser Leu Pro Ile Gly Asn Pro Gln Gly Val Leu Met Ser
                   740                 745                 750
   Ala Ala Glu Gln Ala Val Ser Arg Ile Leu Ala Leu Ala Gly Gly Pro
           755                 760                 765
<210> 11
   <211> 30
   <212> DNA
   <213> Artificial sequence
<220>
   <223> primer
<400> 11
   tatttcgaaa cgatgaggac cacctcggcc 30
<210> 12
   <211> 29
   <212> DNA
   <213> Artificial sequence
<220>
   <223> primer
<400> 12
   tatcacgtgc tacacacact gccaatacc 29

## Description

The field of the present invention relates to recombinant enzyme modification to modify substrate specificity.

Cellobiose dehydrogenase (EC 1.1.99.18, CDH) was first discovered in 1974 in the extracellular enzyme system of *Phanerochaete chrysosporium* and later on in several other basidiomycetous fungi. A special characteristic of this enzyme is its composition: the combination of a catalytically active flavodehydrogenase domain (also called "flavin domain"), hosting a non-covalently bound FAD, and a haem domain, with a haem *b* as a cofactor. Both domains are connected by a linker. By its catalytic activity the natural substrate cellobiose is oxidised in a reaction which reduces the FAD of the flavin domain.

CDH or its flavodehydrogenase domain oxidises carbohydrates like its natural substrates cellobiose and cello-oligosaccharides and others like lactose and maltose. CDHs have been discovered and shown previously to be capable of converting glucose efficiently (Harreither et al., 2011; WO 2010/097462 A). Modified CDHs exist that have reduced activity on maltose (WO2013/131942). Other CDHS are known from *Metarhizium anisopliae* (Database Uniprot, acc. No. E9DZA5) or *Stemphylium lycopersici* (Database Uniprot, acc. No. A0A0L1HDV7). Gao et al. (Plos Genetics, 7 (1), (2001): e1001264) describe genomic sequencing of the fungi *Metarhizium anisopliae* and *M. acridum.* EP 2 636 733 A1 describes artificially mutated CDHs to reduce its maltose oxidation activity.

The problem with CDHs lies in the versatility which diminishes its use in sensors for detecting single analyte in mixtures of several potential substrates. The present invention relates to a modified cellobiose dehydrogenase (CDH) as described in the present claims.

It is a goal of the present disclosure to provide a cellobiose dehydrogenase or its catalytically active flavodehydrogenase domain, which is capable to selectively detect lactose in the presence of glucose and galactose, especially with direct electron transfer based electrodes or mediated electron transfer-based electrodes to provide suitable analytically useful sensors.

The present disclosure relates to recombinant modified cellobiose dehydrogenases (CDH) with reduced glucose and galactose turnover. The aim is to reduce the effect of any glucose and galactose concentration present in a sample matrix on lactose detection. In particular, the disclosure provides a modified cellobiose dehydrogenase (CDH) or its functional flavodehydrogenase domain having a substitution at the amino acid corresponding to N721 of SEQ ID NO: 5 (CDH from *M. thermophilum*) or corresponding to N722 of SEQ ID NO: 4 (CDH from *N. crassa*) by glutamine, isoleucine, threonine or leucine or a substitution of the asparagine in the active center motif having the amino acid sequence NHW by glutamine, isoleucine, threonine or leucine; hence this motif would be QHW, IHW, THW or LHW. Usually, the motif is found near the C-terminus of a CDH or the flavodehydrogenase domain, e.g. around amino acids 660 to 780 of a CDH or amino acids 430 to 550 of a flavodehydrogenase domain. Preferred modified cellobiose dehydrogenase enzymes of the invention are optimized for use in biosensors based on direct electron transfer (3^{rd} generation) or based on mediated electron transfer (2^{nd} generation).

To increase the performance of CDH as selective electrode catalyst for lactose, the reduction of glucose and galactose oxidation activity alone or in combination with an increase of lactose oxidation activity was pursued by genetic modification. With the general description of modifying a CDHs flavodehydrogenase domain, a domain of high homology in all CDHs, it is possible to modify any CDH according to the principles outlined herein in order to decrease glucose and galactose sensitivity. Thus the present invention provides in a particular a modified cellobiose dehydrogenase (CDH) or its functional flavodehydrogenase domain having reduced glucose and galactose oxidation activity as compared to the unmodified CDH or its functional flavodehydrogenase domain while essentially maintaining a high lactose oxidation activity, e.g. at most a 20% reduction in lactose oxidation activity, at standard conditions and as shown in the examples. The invention further provides a method of oxidizing lactose with the CDH of the invention. Such a method is preferably used in the analytical detection of lactose in a sample. Also possible is the detection of maltose.

Most CDHs are capable of oxidizing lactose. The oxidation reaction can be detected e.g. by monitoring electron acceptors for the redox reaction such quinones, like as DCIP (2,6-dichloroindophenol) o- or p-benzoquinone or derivatives thereof, methylene blue, methylene green, Meldola's blue, potassium ferricyanide, ferricenium hexafluorophosphate, FeCl₃ or cytochrome c (the latter being a haem domain cofactor) or simply by determining electric current or voltages on an electrode, the electron acceptor being the electrode surface.

It is not necessary to use an entire CDH; the flavin domain, even without the haem domain, is sufficient for catalytical activity. The domain is therefore referred to as "functional domain" as it has the function of oxidizing lactose with a suitable electron acceptor. The activity is exerted by either the whole enzyme cellobiose dehydrogenase or the catalytically active flavodehydrogenase domain.

Wild type CDHs have an undesirable activity to oxidize glucose and galactose, especially in case of CDHs from ascomycota. The modification according to the present invention should now be understood in that the inventive CDHs deviate from the wild-type CDHs by this substantially decreased glucose and galactose oxidation activity. This substantially decreased glucose and galactose oxidation activity may be in combination with an increased or only small decrease in lactose oxidation activity. In some embodiments, the invention increases the ratio of the lactose oxidation activity to the glucose and/or galactose oxidation activity.

Preferred modified CDHs or their functional flavodehydrogenase domain are of a CDH of the subkingdom *dikarya.* Preferably, the CDH is from Ascomycota or Basidiomycota. Preferred ascomycota are selected from *Myriococcum thermophilum, Corynascus thermophilus, Chaetomium atrobrunneum (Myceliophthora fergusii), Hypoxylon haematostroma, Neurospora crassa* or *Stachybotrys bisby.* Preferred Basidiomycota are *Athelia rolfsii, Gelatoporia subvermispora, Phanerochaete chrysosporium, Trametes versicolor.* Such unmodified CDHs are described in WO 2010/097462 A and in WO2013/131942, including encoding nucleotide sequences, and in sequences of SEQ ID NO: 1-10 herein. Suitable sequences are also available in sequence databases, in particular the NCBI database, e.g. accession numbers ADT70773.1, ADT70775.1, ADT70772.1, XP_956591.1, ABS45567.2, ADT70777.1, AAO64483.1, ACF60617.1, AAB61455.1, XP_008041466.1. "Unmodified" as used herein is a CDH without the inventive modification that decreases glucose and galactose oxidation activity. There may be further modifications that increase interaction between the flavin and the haem domain if an entire CDH is used (such as described in WO 2010/097462).

Preferably the modified CDH or its functional flavodehydrogenase domain is based on an unmodified CDH or flavodehydrogenase domain with an active center motif having the sequence X₁X₂NHWX₃X₄X₅, wherein X₁ is any amino acid, preferably selected from N, C and R; X₂ is selected from S and A; X₃ is selected from V, M and I; X₄ is any amino acid, preferably selected from G and S; and X₅ is any amino acid, preferably selected from S, A and T; especially preferred X₄ and X₅ are not both S. In the inventive modified enzyme or domain, the N in this motif is changed to Q, I, T or L as said above. This change facilitates the inventive activity change reducing activity on glucose and/or galactose. With the information of the motif, the position for the substitution can be determined. Preferably, also the modified CDH or the domain comprises the sequence X₁X₂ZHWX₃X₄X₅, with Z, being Q, I, T or L and X₁-X₅ being defined as above.

The modified CDH or its functional flavodehydrogenase domain preferably comprises a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 233-771 of SEQ ID NO: 7, amino acids 236-774 of SEQ ID NO: 8, amino acids 235-773 of SEQ ID NO: 9, amino acids 230-768 of SEQ ID NO: 10. Preferably the inventive modified flavodehydrogenase domain has a sequence with at least 50 %, preferably at least 55%, at least 60%, at least 65%, at least 70%, at least 75%, at least 80%, at least 85%, at least 90%, at least 95%, at least 98%, in particular preferred at least 99%, sequence identity with one of said unmodified flavodehydrogenase domains and further comprises at least one amino acid substitution at the amino acid corresponding to N721 of SEQ ID NO: 5 or at the NHW motif, suitable for reducing the glucose and galactose oxidation activity.

Preferably, the amino acid corresponding to N721 of SEQ ID NO: 5 or asparagine at the NHW motif is amino acid N723 of SEQ ID NO: 1, N738 of SEQ ID NO: 2, N718 of SEQ ID NO: 3, N722 of SEQ ID NO: 4, N721 of SEQ ID NO: 5, N721 of SEQ ID NO: 6, N704 of SEQ ID NO: 7, N707 of SEQ ID NO: 8, N706 of SEQ ID NO: 9, N701 of SEQ ID NO: 10.

Homologous CDHs or flavodehydrogenase domains within these sequence requirements can be readily identified by sequence comparisons such as by sequence alignment using publicly available tools, such as BLASTP, ClustalW or FastDB. Preferably, a homologous or modified CDH or the domain thereof has 1, 2, 3, 4, 5, 6, 7, 8, 9, 10, at least 11, at last 13, at least 15, at least 17, at least 20, at least 25, at least 30, at least 40, at least 50, at least 60, at least 80, at least 100 and/or up to 100, up to 80, up to 60, up to 50, up to 40, up to 30, up to 30, up to 20, up to 15 further amino acid substitutions, deletions, insertions or modifications, and any ranges between these values, as compared to any one of the CDHs of SEQ ID NOs 1-10 or any of their flavodehydrogenase domains. A particular preferred base CDH is of N. crassa, SEQ ID NO: 4. A "further amino acid substitutions, deletions, insertions" does not include the substitution at the N of N721 corresponding to SEQ ID NO: 5 or at the NHW motif. A prediction of such modifications can be made by computational methods using e.g. molecular docking into crystal structures such as of PDB database entry "1kdg".

The effect of further modified amino acids in the active site and the substrate binding site can be determined for homogeneous catalysis by photometric methods and for heterogeneous catalysis by electrochemical measurements using enzyme electrodes as described herein.

The methods for further modification may be any known in the art such as amino acid mutations, including amino acid substitutions, deletions or additions but also chemical modification/derivatisation of amino acid side chains.

The inventive enzyme or domain is usually recombinantly expressed. Also provided are preparations comprising the modified CDH or domain. The term "enzyme" or "enzyme preparation" as used herein refers to a cellobiose dehydrogenase or its flavodehydrogenase domain from a specified organism which is at least about 20% pure, preferably at least about 40% pure, even more preferably at least about 60% pure, even more preferably at least 80% pure and most preferably at least 90% pure, as determined by polyacrylamide gel electrophoresis (SDS-PAGE) .

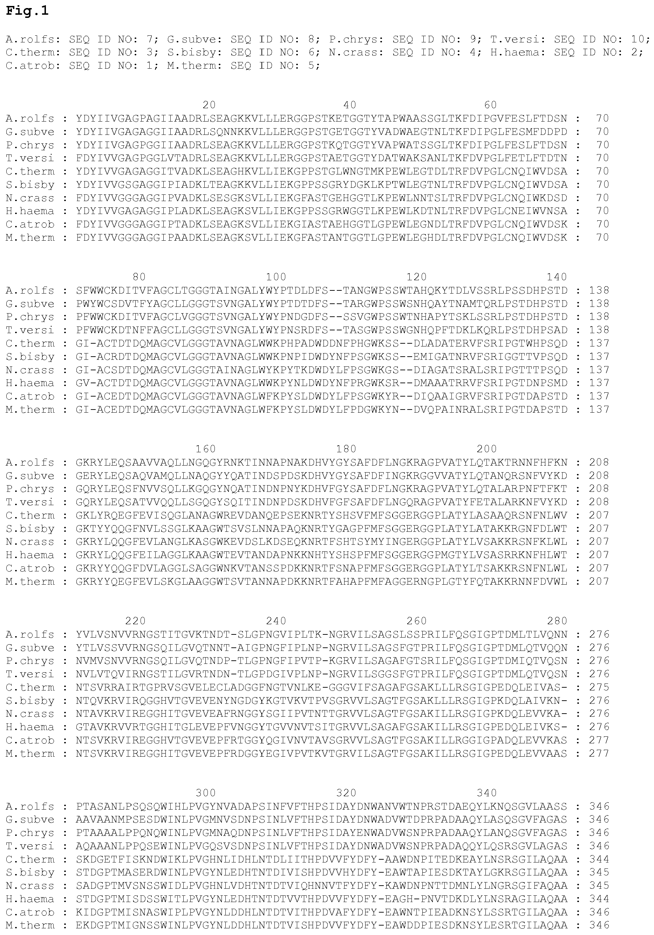

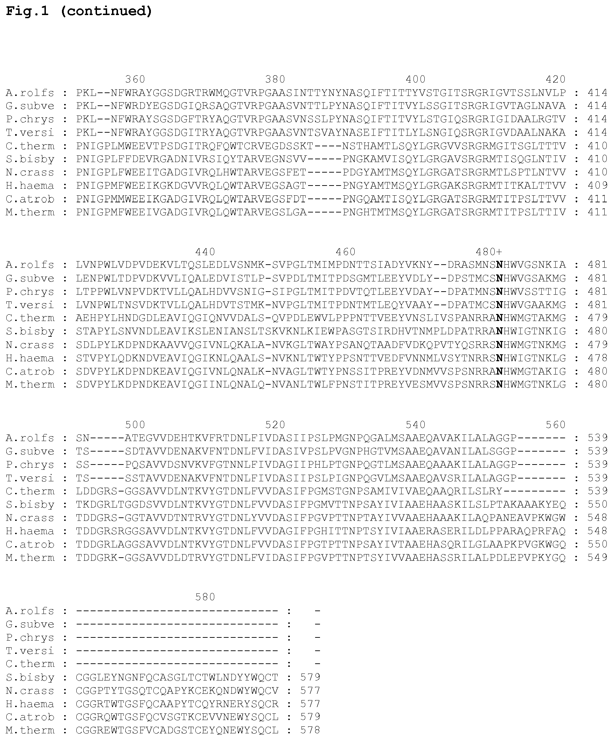

The present disclosure relates to modified/genetically engineered cellobiose dehydrogenases or flavodehydrogenase domain from existing protein scaffolds, which oxidise glucose and/or galactose less efficiently with or without a more efficient lactose turnover than the currently known cellobiose dehydrogenases, especially those that is closest related to the modified CDH or the flavodehydrogenase domain, such as a CDH or flavodehydrogenase domain of sequences SEQ ID NO: 1-10 (the portions for the flavodehydrogenase domain are given below in the description of Fig. 1). The kinetic constants of the enzymes responsible for this effect are preferably a higher K_{M} value and lower k_{cat} value for glucose and/or galactose alone or in combination with a lower K_{M} value and higher k_{cat} value for lactose than the currently characterised enzymes.

Preferably the K_{M} value of the cellobiose dehydrogenase or its functional flavodehydrogenase domain for a lactose oxidation reaction is below 10 mM, preferably as determined with the CDH or said domain being immobilized on an electrode.

Preferably the K_{M} value of the cellobiose dehydrogenase or its functional flavodehydrogenase domain for a glucose and/or galactose oxidation reaction is above 3 M or above 5 M, preferably as determined with the CDH or said domain being immobilized on an electrode.

Of course, the modified CDH or the flavin domain may still have activity for an electrocatalytic oxidation of glucose and/or galactose. It is sufficient that the concentration dependence of the signal from glucose and/or galactose is reduced. Essentially constant background signals dependent on glucose and/or galactose can be removed from the lactose detection by normalization. Especially preferred the CDH or flavin domain has the property that a signal of glucose and/or galactose during lactose detection or lactose concentration determination is below 5%; particularly the signal, e.g. electrode current or electrochemical reduction of an electron acceptor, of 20 g/L glucose and/or galactose in a solution compared to the signal of a 20 g/L lactose solution is below 5%. In preferred embodiments of the invention the glucose and/or galactose oxidation activity of the CDH or the domain is reduced in relation to lactose oxidation activity. Especially preferred the concentration dependency of the glucose and/or galactose oxidation is reduced so that - if glucose and/or galactose is present - only a substantially constant contribution of glucose and/or galactose to the signal is detected in relation to a lactose signal.

It is understood that one of skill in the art may engineer the mentioned or other cellobiose dehydrogenases to obtain the modified CDH or the active flavodehydrogenase domain using the principles outlined herein or in the prior art (WO 2010/097462 A, WO2013/131942) like the rational enzyme engineering via site-directed mutagenesis or directed evolution approaches (e.g., gene shuffling, error-prone PCR, etc.) and subsequent screening of the generated diversity. The techniques to introduce a further mutation into the nucleic acid sequence to exchange one nucleotide for another nucleotide with the aim to exchange one amino acid for another in the resulting protein may be accomplished by site-directed mutagenesis using any of the methods known in the art. The amino acid positions and modification to modify a CDH can be obtained by homology modelling using the crystal structure of *Phanerochaete chrysosporium* CDH (PDB database entry 1kdg) as template and superimposition of the obtained models as well as docking studies. It is possible to use a CDH as template and by sequence comparison to obtain corresponding amino acids for modification to reduce glucose and/or galactose oxidation activity.

The modified CDH or its functional flavodehydrogenase domain may be isolated by diafiltration, ion exchange chromatography and preferably being further purified by hydrophobic interaction chromatography. Preferably the CDH or the domain is recombinantly produced by *Pichia pastoris.*

The invention further provides a nucleic acid molecule encoding a modified CDH or its functional flavodehydrogenase domain as described above. Unmodified CDH sequences are known in the art, e.g. in the NCBI sequence database and disclosed in WO 2010/097462 A and WO2013/131942. The nucleic acid can be modified to encode the inventive CDH or flavin domain with the substitution or further mutation. The nucleic acids, CDH or domains described herein may be isolated and/or purified.

Further provided is a method of producing a modified CDH or its functional flavodehydrogenase domain of the invention, comprising recombinantly expressing a nucleic acid molecule encoding said modified CDH or its functional flavodehydrogenase domain in a host cell.

In a further aspect the invention further provides an electrode comprising an immobilised cellobiose dehydrogenase or its functional flavodehydrogenase domain of the invention.

Preferably the electrode comprises an immobilised CDH in direct- or mediated electron transfer mode (Tasca et al. 2011a, Tasca et al. 2011b, Ludwig et al. 2010, Safina et al. 2010, Tasca et al 2010a, Tasca et al. 2010b) or an immobilised flavodehydrogenase domain in mediated electron transfer mode. As electrode any suitable surface for collecting electrons from CDH is understood. The electrode may be of any material suitable to immobilise the CDH, e.g. carbon such as graphite, pyrolytic graphite, glassy carbon, carbon nanotubes (single or multi-walled), carbon fibres, boron doped diamond, gold electrodes modified with promoters e.g., thiols or screen-printed electrodes. This is a non-exhaustive list of possible electrodes, which may e.g. contain other nanoparticles (gold,...) to increase the specific surface area. Particular uses of the inventive electrodes are in the provision of biosensors, more specifically to lactose biosensors using the direct electron transfer properties (DET) of cellobiose dehydrogenase (CDH) or using mediated electron transfer properties (MET) to measure the lactose concentration at acidic, neutral, alkaline pH.

On the electrode, the CDH or the flavodehydrogenase domain may be immobilised by adsorption, preferably also physical entrapment in a polymer, complex formation, preferably via an additional complexing linker, covalent binding, in particular cross-linking, or ionic binding and/or the immobilized cellobiose dehydrogenase can be cross-linked, in particular by bifunctional agents, to increase stability or activity. It has been shown that cross-linking with bifunctional agents, such as agents with two reactive groups making a connection with the CDH, can stabilize the CDH and even increase its activity on graphite electrodes measurable by amperometric methods described herein. This advantage can lead to an increased sensitivity and lowering the detection limit for lactose. Such a cross-linking agent is e.g. glutaraldehyde or any other dialdehydes. Further methods for immobilization are described in e.g. WO 2010/097462 and WO2013/131942, which can be used according to the invention.

The electrodes might be used in form of a single electrode or electrode stacks, e.g. of 2, 3, 4, or more electrodes.

Electrode configurations and uses, including measurement parameters are described in e.g. WO 2010/097462 and WO2013/131942, which can be used according to the invention.

The disclosure further provides a method of oxidizing lactose with the inventive CDH or flavin domain, especially in a method of detecting or quantifying lactose in a sample comprising the step of oxidizing lactose in said sample with a modified CDH or its functional flavodehydrogenase domain or an electrode as described herein and detecting or quantifying said oxidation, preferably wherein said sample comprises or is suspected of comprising glucose and/or galactose. The fluid sample may be any fluid which potentially comprises lactose, including milk or milk or dairy products, such as whey or cheese. If solid products are analysed, such as cheese, the carbohydrates, including lactose, may be extracted or the solid product may be fluidized, such as by dissolving.

In a further aspect the present invention provides a lactose assay kit comprising the modified cellobiose dehydrogenase or its functional flavodehydrogenase domain or an electrode as described herein. The kit may in preferred embodiments also comprise auxiliary substances, like buffers, and containers such as a sample holding means and/or lactose standards. Lactose standards may be used to calibrate the assay. The kit may also comprise a reader for a signal, especially an electrochemical signal such as a potentiostat, a computer readable memory device with software for calibration and/or measurement calculations. The invention can be defined by the following embodiments:
1. A modified cellobiose dehydrogenase (CDH) or its functional flavodehydrogenase domain having a substitution at the amino acid corresponding to N721 of SEQ ID NO: 5 (CDH from *M. thermophilum*) by glutamine, isoleucine, threonine or leucine or a substitution of the asparagine in the active canter motif having the amino acid sequence NHW by glutamine, isoleucine, threonine or leucine.
2. The modified CDH or its functional flavodehydrogenase domain according to 1, wherein active center motif has the sequence X₁X₂NHWX₃X₄X₅, wherein X₁ is any amino acid, preferably selected from N, C and R; X₂ is selected from S and A; X₃ is selected from V, M and I; X₄ is any amino acid, preferably selected from G and S; and X₅ is any amino acid, preferably selected from S, A and T; especially preferred X₄ and X₅ are not both S.
3. The modified CDH or its functional flavodehydrogenase domain according to 1 or 2, characterised in that the CDH is of *dikary*ota, preferably selected from *ascomycota* or *basidiomycota.*
4. The modified CDH or its functional flavodehydrogenase domain according to 3, characterized in that the CDH is a modified CDH of a CDH from *Myriococcum thermophilum, Corynascus thermophilus, Chaetomium atrobrunneum (Myceliophthora fergusii), Hypoxylon haematostroma, Neurospora crassa* or *Stachybotrys bisby, Athelia rolfsii, Gelatoporia subvermispora, Phanerochaete chrysosporium, Trametes versicolor.*
5. The modified CDH or its functional flavodehydrogenase domain according to any one of 1 to 4, comprising a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 233-771 of SEQ ID NO: 7, amino acids 236-774 of SEQ ID NO: 8, amino acids 235-773 of SEQ ID NO: 9, amino acids 230-768 of SEQ ID NO: 10;
   said modified flavodehydrogenase domain having a sequence with at least 50 %, preferably at least 55%, at least 60%, at least 65%, at least 70%, at least 75%, at least 80%, at least 85%, at least 90%, at least 95%, at least 98%, in particular preferred at least 99%, sequence identity with one of said unmodified flavodehydrogenase domains and further comprises the substitution mutation at amino acid corresponding to N721 of SEQ ID NO: 5 or at the NHW motif.
6. The modified CDH or its functional flavodehydrogenase domain according to any one of 1 to 5, wherein the amino acid corresponding to N721 of SEQ ID NO: 5 or asparagine at the NHW motif is amino acid N723 of SEQ ID NO: 1, N738 of SEQ ID NO: 2, N718 of SEQ ID NO: 3, N722 of SEQ ID NO: 4, N721 of SEQ ID NO: 5, N721 of SEQ ID NO: 6, N704 of SEQ ID NO: 7, N707 of SEQ ID NO: 8, N706 of SEQ ID NO: 9, N701 of SEQ ID NO: 10.
7. The modified CDH or its functional flavodehydrogenase domain according to any one of 1 to 6, being recombinantly produced by *Pichia pastoris,* isolated by diafiltration, ion exchange chromatography and preferably being further purified by hydrophobic interaction chromatography.
8. The modified CDH or its functional flavodehydrogenase domain according to any one of 1 to 7, characterised in that the K_{M} value of the cellobiose dehydrogenase or its functional flavodehydrogenase domain for a lactose oxidation reaction is below 10 mM, preferably as determined with the CDH or said domain being immobilized on an electrode.
9. The modified CDH or its functional flavodehydrogenase domain according to any one of 1 to 8, characterised in that the K_{M} value of the cellobiose dehydrogenase or its functional flavodehydrogenase domain for a glucose oxidation reaction is above 3 M, preferably as determined with the CDH or said domain being immobilized on an electrode.
10. The modified CDH or its functional flavodehydrogenase domain according to any one of 1 to 9, characterised in that the K_{M} value of the cellobiose dehydrogenase or its functional flavodehydrogenase domain for a galactose oxidation reaction is above 3 M, preferably as determined with the CDH or said domain being immobilized on an electrode.
11. A nucleic acid molecule encoding a modified CDH or its functional flavodehydrogenase domain according to any one of 1 to 10.
12. A method of producing a modified CDH or its functional flavodehydrogenase domain according to any one of 1 to 10, comprising recombinantly expressing a nucleic acid molecule according to 11 in a host cell.
13. An electrode comprising an immobilised cellobiose dehydrogenase or its functional flavodehydrogenase domain according to any one of 1 to 10;
   preferably wherein the cellobiose dehydrogenase is immobilised by adsorption, complex formation, especially preferred via an additional complexing linker, covalent or ionic linkage, and/or wherein preferably the immobilized cellobiose dehydrogenase is cross-linked, in particular by bifunctional agents, to increase stability or activity.
14. Method of detecting or quantifying lactose in a sample comprising the step of oxidizing lactose in said sample with a modified CDH or its functional flavodehydrogenase domain according to any one of 1 to 10, or an electrode according to 13 and detecting or quantifying said oxidation, preferably wherein said sample comprises or is suspected of comprising glucose and/or galactose, especially preferred a milk or milk product containing sample.
15. A lactose assay kit comprising the modified Cellobiose dehydrogenase or its functional flavodehydrogenase domain according to any of the 1 to 10 or an electrode according to 13 and a sample holding means and/or lactose standards.

The present invention is further illustrated by the following figures and examples without being restricted thereto.

### Figures:

**Figure** 1 is a sequence alignment of amino acid sequences of the flavodehydrogenase domains ("flavin domains") of the wild-type CDHs from *Chaetomium atrobrunneum* (aa 253-831 of SEQ ID NO: *1), Hypoxylon haematostroma* (aa 269-845 of SEQ ID NO: 2), *Corynascus thermophilus* (aa 249-787 of SEQ ID NO: 3), *Neurospora crassa* (aa 253-829 of SEQ ID NO: 4), *Myriococcum thermophilum* (aa 251-828 of SEQ ID NO: 5), *Stachybotrys bisby* (aa 251-829 of SEQ ID NO: 6), *Athelia rolfsii* (aa 233-771 of SEQ ID NO: 7), *Gelatoporia subvermispora* (aa 236-774 of SEQ ID NO: 8), *Phanerochaete chrysosporium* (aa 235-773 of SEQ ID NO: 9), *Trametes versicolor* (aa 230-768 of SEQ ID NO: 10). The mutation site at corresponding to N721 of SEQ ID NO: 5 is highlighted and marked by "+" (position 483 of the shown consensus numbering of the shown flavin domains).
**Figure 2** provides lactose calibration curves of sensor electrodes featuring unmodified wild-type *N. crassa* CDH and *N. crassa* N722Q CDH.
**Figure 3** shows the effect of spiking of a 0.1 g lactose solution with different concentrations of glucose and galactose when using unmodified wild-type *N. crassa* CDH and *N. crassa* N722Q CDH. The signal for the unmodified wild-type *N. crassa* CDH is glucose and galactose dependent. The signal for the *N. crassa* N722Q CDH is not glucose and galactose dependent.
**Figure 4** provides glucose calibration curves of sensor electrodes featuring unmodified wild-type *N. crassa* CDH and *N. crassa* CDH variant N722Q CDH.
**Figure 5** provides galactose calibration curves of sensor electrodes featuring unmodified wild-type *N. crassa* CDH and *N. crassa* CDH variant N722Q CDH.

### Examples:

### Example 1: Materials

Chemicals used in buffers and fermentation media were commercial products and at least of analytical grade if not otherwise stated. Substrates for kinetic studies were lactose, galactose, glucose and 2,6-dichloroindophenol (DCIP) from Sigma-Aldrich in the highest grade of purity available. Buffers were prepared using water purified and deionised (18 MΩ) with a Milli-Q system (Millipore, Bedford, MA, USA).

### Example 2: Enzymatic activity assays and steady-state kinetics

Enzyme activity was assayed at 30°C using the DCIP (Karapetyan et al., 2005 Journal of Biotechnology 121: 34-48) as electron acceptor. Stock solutions of carbohydrates used for kinetic measurements were prepared in the respective buffer and allowed to stand overnight for mutarotation, while stock solutions of electron acceptors were prepared in water and immediately used. The reaction stoichiometry is 1 for the two-electron acceptor DCIP (1 mole of DCIP reduced per mole of carbohydrate oxidized. Kinetic constants were calculated by fitting the observed data to the Henri-Michaelis-Menten equation or to the adapted model for substrate inhibition using nonlinear least-squares regression and the program SigmaPlot (Systat Software, San Jose, CF, USA). The protein concentration was determined with the Bradford assay.

### Example 3: Protein characterisation

The protein concentration was determined by the dye-staining method of Bradford using a pre-fabricated assay from Bio-Rad Laboratories Hercules, CA, USA) and bovine serum albumin as standard according to the manufacturers recommendations.
For electrophoretic characterisation SDS-PAGE was carried out on a Hoefer SE 260 Mighty Small II vertical electrophoresis unit. Gels (10.5x10 cm; 10% T, 2.7% C) were cast and run according to the manufacturers' modifications of the Laemmli system. Protein bands on the SDS-PAGE were stained with silver, bands on the IEF gel with Coomassie blue R-250, according to the instructions.

### Example 4: Lactose to glucose/galactose activity

Amino acid N721 of the *M. therm.* CDH which is part of the catalytic subsite, close to the isoalloxazine ring and fully conserved among CDHs was selected for saturation mutagenesis. Degenerated codons of the NNS type were used. The corresponding mutant library was transformed into *S. cerevisiae* and expressed under the control of the GAL 1 promotor in four 96 well plates. Each plate contained 88 variants and 8 wells were inoculated with the wild type as control. The libraries were double screened for CDH activity with the DCIP-based assay using lactose or glucose as substrate. 11 Transformants which showed an increased ratio of lactose to glucose activity were sent for sequencing to identify the beneficial mutation. Table 1 shows the results of the screening and the identified amino acid exchanges. The selected variants showed an increase in lactose activity while the glucose activity was dramatically reduced compared to the wild type.

**Table 1: Lactose to glucose/galactose activity**

| | DCIP act. (ΔAbs. h⁻¹) ratio | | | |
|---|---|---|---|---|
| | 30 mM Lactose | 50 mM Glucose | Lactose/ Glucose | Amino acid exchange |
| *Wt (av.)* | 1.7 | 0.27 | 6.3 | none |
| Plate 1 D05 | 4.7 | 0.03 | 156 | Q |
| Plate 1 G11 | 4.6 | 0.02 | 230 | Q |
| Plate 2 B05 | 3.8 | 0.02 | 190 | I |
| Plate 2 E05 | 5.9 | 0.01 | 590 | I |
| Plate 2 G05 | 3.8 | 0.01 | 380 | T |
| Plate 2 F07 | 3.8 | 0.04 | 95 | T |
| Plate 3 A11 | 5.0 | 0.11 | 45 | Q |
| Plate 3 C10 | 4.8 | 0.02 | 240 | L |
| Plate 3 H11 | 5.6 | 0.02 | 280 | Q |
| Plate 4 G08 | 4.7 | 0.02 | 235 | Q |
| Plate 4 G10 | 4.7 | 0.01 | 470 | Q |

Activity on galactose resembled the results on glucose.

### Example 5: Generation of Neurospora crassa CDH variants by site-directed mutagenesis

The previously reported plasmid pNCIIA, encoding the *N. crassa* CDH gene (XM_951498, SEQ ID NO: 12 of WO 2010/097462, incorporated herein by reference) was used as templates for the amplification of *the target gene* with primers 5NCa-BstBI (5'-TATTTCGAAACGATGAGGACCACCTCGGCC-3', SEQ ID NO: 11) and 3NCa-XbaI (5'-TATCACGTGCTACACACACTGCCAATACC-3', SEQ ID NO: 12). PCR was performed with Phusion high-fidelity DNA polymerase from New England BioLabs, a deoxynucleoside triphosphate (dNTP) mix from Fermentas, oligonucleotide primers from VBC Biotech (Vienna, Austria), and a C-1000 thermocycler from Bio-Rad Laboratories. The resulting PCR fragment was digested with BstBI and XbaI and cloned into the equally treated vector pPICZα A. The procedure resulted in a gene encoding a protein with its native signal sequences cloned under the control of the methanol-inducible AOX1 promoter. C-terminal tags for purification or antibody detection were omitted. The correct insertion of the genes and the absence of mutations were confirmed by DNA sequencing. Linearized, verified plasmids were used for transformation into electrocompetent *P. pastoris* cells and transformants were selected on YPD Zeocin plates (1mg L⁻¹).

### Example 6: Production of recombinant CDH

Recombinant wild-type CDH as well as the variant were produced in 1L baffled flasks. Precultures were grown overnight in 30 mL of YPD medium at 30°C and 120 rpm. After approximately 18 hours the precultures were transferred into 1L baffled flasks containing 200 mL BMGY medium without methanol. Induction with methanol was started immediately using a multichannel peristaltic pump (Minipuls Evolution, Gilson, Middleton, WI, USA). Each flask was supplied eight times a day with methanol yielding a total concentration of 2% (v/v) methanol per day. Increase in activity was monitored using the DCIP and the cytochrome c enzyme assays. Cultivation was stopped at day five of methanol induction, cells removed by centrifugation (4000 rpm, 20 min) and the supernatant set to a final ammonium sulfate concentration of 20%.

### Example 7: Purification of recombinant CDH

Enzymes were purified to homogeneity in a two-step purification. The sample was loaded on a 20 mL PHE Sepharose FF column (HR26/20) equilibrated with 50 mM Na-acetate buffer pH 5.5 containing 20% ammonium sulfate. Proteins were eluted by increasing the concentration of the elution buffer (50 mM Na-acetate buffer pH 5.5) from 0 to 100% in 5 column volumes and fractions containing CDH activity were pooled. After diafiltration with a polyethersulfone flat-stack cross flow module with a cut-off of 10 kDa (Viva Flow 50, Sartorius, Göttingen, Germany) until conductivity of 5 mS cm⁻¹ in 20 mM Na-acetate pH 5.5 the samples were loaded on a 20 mL Q-Source column (HR26/20) equilibrated with a 20 mM Na-acetate buffer pH 5.5. CDH was eluted by increasing the concentration of the elution buffer (50 mM Na-acetate buffer pH 5.5 containing 0.5 M NaCl) from 0 to 100% in 50 column volumes. Fractions were tested for CDH activity and pooled according to the highest Reinheitszahl (RZ, calculated from the absorbance ratio 420 nm/280 nm). Purified enzymes were concentrated and diafiltered in 50 mM citrate buffer, pH 5.5, aliquoted and kept at 4°C for further use.

### Example 8: Electrochemical Measurements

A screen printed electrode with a three electrode setup (Dropsens, Oviedo, Spain) was connected to an Autolab potentiostat (PGSTAT204, Metrohm Autolab B. V., Utrecht, The Netherlands) and used for electrochemical measurements. The enzyme modified, screen printed electrodes (DropSens, Oviedo, Spain) were mounted using an electrode holder (DropSens, Oviedo, Spain) into a beaker filled with 50 ml of measuring buffer and optionally varying concentrations of glucose. The system was controlled by the NOVA software version 1.11.0 (Metrohm Autolab B. V., Utrecht, The Netherlands).

### Example 9: Mutated CDH from N. crassa - reduced glucose, galactose activity

CDH from *N. crassa* oxidises glucose and galactose (Harreither et al., 2007), which has negative side effects on the lactose detection accuracy if glucose and galactose are present. To reduce the activity with glucose and galactose, *N. crassa* CDH was used as a protein scaffold for which the glucose and galactose activities were greatly reduced. The enzyme variant N722Q was heterologously produced in *P. pastoris* according to the explained routines and compared to the recombinant wild-type CDH from *N. crassa.* The molecular weights did not differ significantly from the native enzyme produced by the fungus. To investigate the effect of the mutation in the *N. crassa* variant a full characterization of steady-state constants was performed (Tables 2 and 3).

**Table 2: Apparent kinetic constants of wtCDH for indicated electron donors in 100 mM McIlvaine buffer at pH 5.5 using DCIP as artificial electron acceptor**

| Enzyme | Substrate (Electron donors) | Mean Kₘ (mM) ± SD | Mean Vₘₐₓ (U/mg) ± SD | Mean k_{cat} (s⁻¹) | Mean k_{cat}/Kₘ (mM⁻¹ s⁻¹) |
|---|---|---|---|---|---|
| wtCDH | Lactose | 0.154 ± 0.01 | 18.2 ± 0.56 | 25.78 | 167.4 |
| | Glucose^{a} | 2039 ± 0.41 | 20.3 ± 2.34 | 28.76 | 12.03 |
| | Galactose^{a} | 26.5 ± 6.85 | 6.33 ± 1.57 | 8.97 | 3.75 |

| | | | | | |
|---|---|---|---|---|---|
| ^{a} Extrapolated Km and kcat values. | | | | | |

**Table 3: Apparent kinetic constants of N722Q N. crassa sensor for indicated electron donors in 100 mM McIlvaine buffer at pH 5.5 using DCIP as artificial electron acceptor**

| Enzyme | Substrate (Electron donors) | Mean Kₘ (mM) ± SD | Mean Vₘₐₓ (U/mg) ± SD | Mean k_{cat} (s⁻¹) | Mean k_{cat}/Kₘ (mM⁻¹ s⁻¹) |
|---|---|---|---|---|---|
| N722Q | Lactose Glucose^{a} | 2.18 ± 0.28 > 10 M^{b} | 55.0 ± 2.0 | 77.92 | 35.74 |
| | Galactose^{a} | > 10 M ^{b} | Data could not be calculated^{b} | | |

| | | | | | |
|---|---|---|---|---|---|
| ^{a} Extrapolated Km and kcat values. ^{b} Data could not be analyzed as it was still located within linear range. | | | | | |

The constants in Table 3 show in more detail that glucose and galactose oxidation activity is suppressed in *N. crassa* CDH variant.

### Example 10: Mutated CDH from N. crassa - Electrode performance

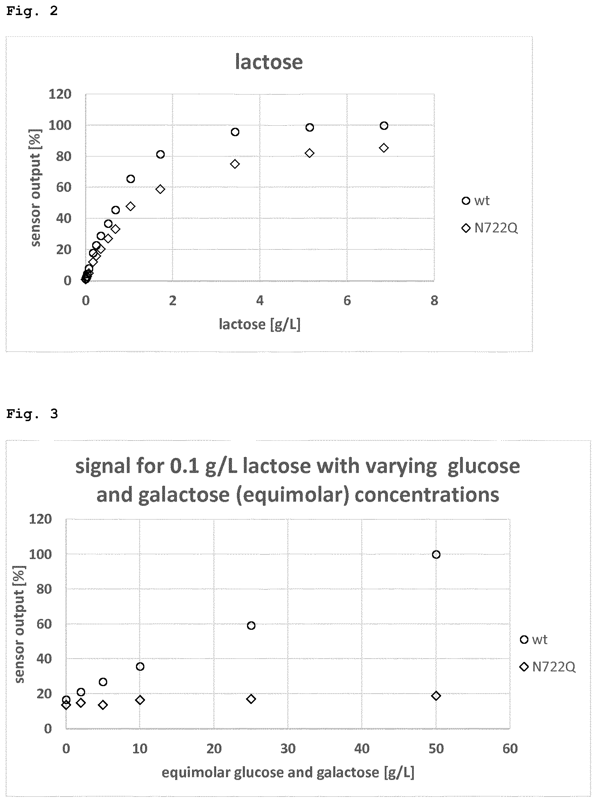

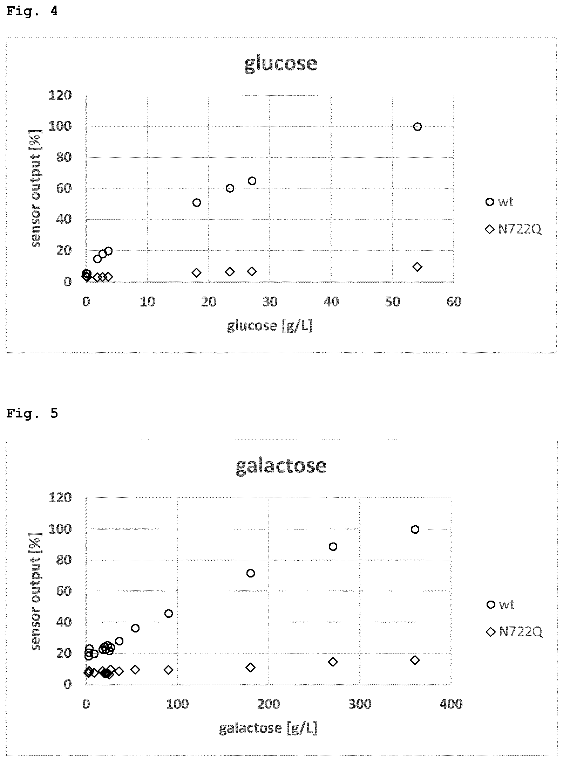

To test the modified *N. crassa* CDH variant was immobilized on graphite electrode and the current responses in different concentrations of glucose, galactose and lactose were measured. Figures 2-6 show the influence of different glucose or galactose concentrations on lactose measurements. In contrast to the wild-type CDH, no significant influence of glucose and galactose on lactose measurements could be detected in the measurements with the modified CDH variants.

### References

Gao et al. (2001) Plos Genetics, 7 (1): e1001264
Harreither et al. (2011) Appl. Environ. Microbiol. 77:1804-1815.
Ludwig et al. (2010) Chem. Phys. Chem. 11:2674-2697
Safina et al. (2010) Electrochimica Acta 55: 7690-7695.
Tasca et al. (2010) Bioelect. 25:1710-1716.
Tasca et al. (2010b) Bioelect. 25:1710-1716.
Tasca et al. (2011) Anal. Chem. 83:3042-3049.
Tasca et al. (2011b). Analyst 136:2033-2036.

## Claims

1. A modified cellobiose dehydrogenase (CDH) or its functional flavodehydrogenase domain having a substitution at the amino acid corresponding to N721 of SEQ ID NO: 5 (CDH from *M. thermophilum*) by glutamine, isoleucine, threonine or leucine or a substitution of the asparagine in the active center motif having the amino acid sequence NHW by glutamine, isoleucine, threonine or leucine,
comprising a modified flavodehydrogenase domain based on one of the unmodified flavodehydrogenase domains according to amino acids 253-831 of SEQ ID NO: 1, amino acids 269-845 of SEQ ID NO: 2, amino acids 249-787 of SEQ ID NO: 3, amino acids 253-829 of SEQ ID NO: 4, amino acids 251-828 of SEQ ID NO: 5, amino acids 251-829 of SEQ ID NO: 6, amino acids 233-771 of SEQ ID NO: 7, amino acids 236-774 of SEQ ID NO: 8, amino acids 235-773 of SEQ ID NO: 9, amino acids 230-768 of SEQ ID NO: 10; said modified flavodehydrogenase domain having a sequence with at least 50% sequence identity with one of said unmodified flavodehydrogenase domains and further comprises the substitution mutation at amino acid corresponding to N721 of SEQ ID NO: 5 or at the NHW motif.

2. The modified CDH or its functional flavodehydrogenase domain according to claim 1, wherein active center motif has the sequence X₁X₂NHWX₃X₄X₅, wherein X₁ is any amino acid, preferably selected from N, C and R; X₂ is selected from S and A; X₃ is selected from V, M and I; X₄ is any amino acid, preferably selected from G and S; and X₅ is any amino acid, preferably selected from S, A and T; especially preferred X₄ and X₅ are not both S.

3. The modified CDH or its functional flavodehydrogenase domain according to claim 1 or 2, **characterised in that** the CDH is of *dikaryota,* preferably selected from *ascomycota* or *basidiomycota.*

4. The modified CDH or its functional flavodehydrogenase domain according to claim 3, **characterized in that** the CDH is a modified CDH of a CDH from *Myriococcum thermophilum, Corynascus thermophilus, Chaetomium atrobrunneum (Myceliophthora fergusii), Hypoxylon haematostroma, Neurospora crassa* or *Stachybotrys bisby, Athelia rolfsii, Gelatoporia subvermispora, Phanerochaete chrysosporium, Trametes versicolor.*

5. The modified CDH or its functional flavodehydrogenase domain according to any one of claims 1 to 4, wherein said modified flavodehydrogenase domain has a sequence with at least 70%, preferably at least 75%, at least 80%, at least 85%, at least 90%, at least 95%, at least 98%, in particular preferred at least 99%, sequence identity with one of said unmodified flavodehydrogenase domains and further comprises the substitution mutation at amino acid corresponding to N721 of SEQ ID NO: 5 or at the NHW motif.

6. The modified CDH or its functional flavodehydrogenase domain according to any one of claims 1 to 5, wherein the amino acid corresponding to N721 of SEQ ID NO: 5 or asparagine at the NHW motif is amino acid N723 of SEQ ID NO: 1, N738 of SEQ ID NO: 2, N718 of SEQ ID NO: 3, N722 of SEQ ID NO: 4, N721 of SEQ ID NO: 5, N721 of SEQ ID NO: 6, N704 of SEQ ID NO: 7, N707 of SEQ ID NO: 8, N706 of SEQ ID NO: 9, N701 of SEQ ID NO: 10.

7. The modified CDH or its functional flavodehydrogenase domain according to any one of claims 1 to 6, being recombinantly produced by *Pichia pastoris,* isolated by diafiltration, ion exchange chromatography and preferably being further purified by hydrophobic interaction chromatography.

8. The modified CDH or its functional flavodehydrogenase domain according to any one of claims 1 to 7, **characterised in that** the K_{M} value of the cellobiose dehydrogenase or its functional flavodehydrogenase domain for a lactose oxidation reaction is below 10 mM, preferably as determined with the CDH or said domain being immobilized on an electrode.

9. The modified CDH or its functional flavodehydrogenase domain according to any one of claims 1 to 8, **characterised in that** the K_{M} value of the cellobiose dehydrogenase or its functional flavodehydrogenase domain for a glucose oxidation reaction is above 3 M, preferably as determined with the CDH or said domain being immobilized on an electrode.

10. The modified CDH or its functional flavodehydrogenase domain according to any one of claims 1 to 9, **characterised in that** the K_{M} value of the cellobiose dehydrogenase or its functional flavodehydrogenase domain for a galactose oxidation reaction is above 3 M, preferably as determined with the CDH or said domain being immobilized on an electrode.

11. A nucleic acid molecule encoding a modified CDH or its functional flavodehydrogenase domain according to any one of claims 1 to 10.

12. A method of producing a modified CDH or its functional flavodehydrogenase domain according to any one of claims 1 to 10, comprising recombinantly expressing a nucleic acid molecule according to claim 11 in a host cell.

13. An electrode comprising an immobilised cellobiose dehydrogenase or its functional flavodehydrogenase domain according to any one of claims 1 to 10;
preferably wherein the cellobiose dehydrogenase is immobilised by adsorption, complex formation, especially preferred via an additional complexing linker, covalent or ionic linkage, and/or wherein preferably the immobilized cellobiose dehydrogenase is cross-linked, in particular by bifunctional agents, to increase stability or activity.

14. Method of detecting or quantifying lactose in a sample comprising the step of oxidizing lactose in said sample with a modified CDH or its functional flavodehydrogenase domain according to any one of claims 1 to 10, or an electrode according to claim 13 and detecting or quantifying said oxidation, preferably wherein said sample comprises or is suspected of comprising glucose and/or galactose, especially preferred a milk or milk product containing sample.

15. A lactose assay kit comprising the modified Cellobiose dehydrogenase or its functional flavodehydrogenase domain according to any of the claims 1 to 10 or an electrode according to claim 13 and a sample holding means and/or lactose standards.

## Patentansprüche

1. Modifizierte Cellobiosedehydrogenase (CDH) oder funktionelle Flavodehydrogenasedomäne von dieser mit einer Substitution an der Aminosäure, die N721 gemäß SEQ ID NR: 5 entspricht (CDH aus *M. Thermophilum*), durch Glutamin, Isoleucin, Threonin oder Leucin oder mit einer Substitution des Asparagins in dem aktiven Zentrum-Motiv mit der Aminosäuresequenz NHW durch Glutamin, Isoleucin, Threonin oder Leucin,
umfassend eine modifizierte Flavodehydrogenasedomäne basierend auf einer der unmodifizierten Flavodehydrogenasedomänen gemäß der Aminosäuren 253 bis 831 von SEQ ID NR: 1, der Aminosäuren 269 bis 845 von SEQ ID NR: 2, der Aminosäuren 249 bis 787 von SEQ ID NR: 3, der Aminosäuren 253 bis 829 von SEQ ID NR: 4, der Aminosäuren 251 bis 828 von SEQ ID NR: 5, der Aminosäuren 251 bis 829 von SEQ ID NR: 6, der Aminosäuren 233 bis 771 von SEQ ID NR: 7, der Aminosäuren 236 bis 774 von SEQ ID NR: 8, der Aminosäuren 235 bis 773 von SEQ ID NR: 9 oder der Aminosäuren 230 bis 768 von SEQ ID NR: 10; wobei die modifizierte Flavodehydrogenasedomäne über eine Sequenz verfügt, die zu mindestens 50 % identisch mit einer der unmodifizierten Flavodehydrogenasedomänen ist und des Weiteren die Substitutionsmutation an der Aminosäure, die N721 gemäß SEQ ID NR: 5 entspricht, oder an dem NHW-Motiv umfasst.

2. Modifizierte CDH oder funktionelle Flavodehydrogenasedomäne von dieser nach Anspruch 1, wobei das aktive Zentrum-Motiv die Sequenz X₁X₂NHWX₃X₄X₅ aufweist, wobei X₁ für eine beliebige Aminosäure steht, die bevorzugt aus N, C und R ausgewählt ist; X₂ aus S und A ausgewählt ist; X₃ aus V, M und I ausgewählt ist; X₄ für eine beliebige Aminosäure steht, die bevorzugt aus G und S ausgewählt ist; und X₅ für eine beliebige Aminosäure steht, die bevorzugt aus S, A und T ausgewählt ist; wobei insbesondere bevorzugt X₄ und X₅ nicht beide für S stehen.

3. Modifizierte CDH oder funktionelle Flavodehydrogenasedomäne von dieser nach Anspruch 1 oder 2, **dadurch gekennzeichnet, dass** die CDH von Dikaryonten stammt, die bevorzugt aus *Ascomycota* oder *Basidiomycota* ausgewählt sind.

4. Modifizierte CDH oder funktionelle Flavodehydrogenasedomäne von dieser nach Anspruch 3, **dadurch gekennzeichnet, dass** es sich bei der CDH um eine modifizierte CDH einer CDH aus *Myriococcum thermophilum, Corynascus thermophilus, Chaetomium atrobrunneum (Myceliophthora fergusii), Hypoxylon haematostroma, Neurospora crassa* oder *Stachybotrys bisby, Athelia rolfsii, Gelatoporia subvermispora, Phanerochaete chrysosporium* oder *Trametes versicolor* handelt.

5. Modifizierte CDH oder funktionelle Flavodehydrogenasedomäne von dieser nach einem der Ansprüche 1 bis 4, wobei die modifizierte Flavodehydrogenasedomäne eine Sequenz aufweist, die zu mindestens 70%, bevorzugt zu mindestens 75%, zu mindestens 80%, zu mindestens 85%, zu mindestens 90%, zu mindestens 95%, zu mindestens 98%, insbesondere bevorzugt zu mindestens 99% identisch mit einer der unmodifizierten Flavodehydrogenasedomänen ist und des Weiteren die Substitutionsmutation an der Aminosäure, die N721 gemäß SEQ ID NR: 5 entspricht, oder an dem NHW-Motiv umfasst.

6. Modifizierte CDH oder funktionelle Flavodehydrogenasedomäne von dieser nach einem der Ansprüche 1 bis 5, wobei es sich bei der Aminosäure, die N721 gemäß SEQ ID NR: 5 entspricht, oder bei dem Asparagin an dem NHW-Motiv um die Aminosäure N723 gemäß SEQ ID NR: 1, N738 gemäß SEQ ID NR: 2, N718 gemäß SEQ ID NR: 3, N722 gemäß SEQ ID NR: 4, N721 gemäß SEQ ID NR: 5, N721 gemäß SEQ ID NR: 6, N704 gemäß SEQ ID NR: 7, N707 gemäß SEQ ID NR: 8, N706 gemäß SEQ ID NR: 9 oder N701 gemäß SEQ ID NR: 10 handelt.

7. Modifizierte CDH oder funktionelle Flavodehydrogenasedomäne von dieser nach einem der Ansprüche 1 bis 6, rekombinant produziert durch *Pichia pastoris,* isoliert mittels Diafiltration bzw. Ionenaustauschchromatographie und bevorzugt weiter aufgereinigt mittels hydrophober Interaktionschromatographie.

8. Modifizierte CDH oder funktionelle Flavodehydrogenasedomäne von dieser nach einem der Ansprüche 1 bis 7, **dadurch gekennzeichnet, dass** der K_{M}-Wert der Cellobiosedehydrogenase oder der funktionellen Flavodehydrogenasedomäne von dieser für eine Lactose-Oxidationsreaktion weniger als 10 mM beträgt, was bevorzugt unter Immobilisierung der CDH oder der Domäne auf einer Elektrode bestimmt wird.

9. Modifizierte CDH oder funktionelle Flavodehydrogenasedomäne von dieser nach einem der Ansprüche 1 bis 8, **dadurch gekennzeichnet, dass** der K_{M}-Wert der Cellobiosedehydrogenase oder der funktionellen Flavodehydrogenasedomäne von dieser für eine Glucose-Oxidationsreaktion mehr als 3 M beträgt, was bevorzugt unter Immobilisierung der CDH oder der Domäne auf einer Elektrode bestimmt wird.

10. Modifizierte CDH oder funktionelle Flavodehydrogenasedomäne von dieser nach einem der Ansprüche 1 bis 9, **dadurch gekennzeichnet, dass** der K_{M}-Wert der Cellobiosedehydrogenase oder der funktionellen Flavodehydrogenasedomäne von dieser für eine Galactose-Oxidationsreaktion mehr als 3 M beträgt, was bevorzugt unter Immobilisierung der CDH oder der Domäne auf einer Elektrode bestimmt wird.

11. Nukleinsäuremolekül, das für eine modifizierte CDH oder eine funktionelle Flavodehydrogenasedomäne von dieser nach einem der Ansprüche 1 bis 10 kodiert.

12. Verfahren zur Herstellung einer modifizierten CDH oder einer funktionellen Flavodehydrogenasedomäne von dieser nach einem der Ansprüche 1 bis 10, umfassend die in einer Wirtszelle erfolgende, rekombinante Expression eines Nukleinsäuremoleküls nach Anspruch 11.

13. Elektrode, umfassend eine immobilisierte Cellobiosedehydrogenase oder eine immobilisierte funktionelle Flavodehydrogenasedomäne von dieser nach einem der Ansprüche 1 bis 10;
wobei die Immobilisierung der Cellobiosedehydrogenase bevorzugt mittels Adsorption, Komplexbildung, insbesondere bevorzugt über einen zusätzlichen Komplexierungslinker, oder kovalente oder ionische Verknüpfung erfolgt und/oder wobei die immobilisierte Cellobiosedehydrogenase zur Erhöhung von deren Stabilität oder Aktivität bevorzugt vernetzt ist, insbesondere anhand von bifunktionellen Agenzien.

14. Verfahren zur Detektion oder Quantifizierung von Lactose in einer Probe, umfassend den Schritt des Oxidierens der in der Probe vorliegenden Lactose mit einer modifizierten CDH oder einer funktionellen Flavodehydrogenasedomäne von dieser nach einem der Ansprüche 1 bis 10 oder einer Elektrode nach Anspruch 13 sowie des Detektierens oder Quantifizierens der Oxidation, wobei die Probe bevorzugt Glucose und/oder Galactose umfasst oder mutmaßlich umfasst und es sich insbesondere bevorzugt um eine Probe handelt, die Milch oder ein Milchprodukt enthält.

15. Lactose-Test-Kit, umfassend die modifizierte Cellobiosedehydrogenase oder die funktionelle Flavodehydrogenasedomäne von dieser nach einem der Ansprüche 1 bis 10 oder eine Elektrode nach Anspruch 13 sowie ein Mittel zur Probenaufnahme und/oder Lactose-Standards.

## Revendications

1. Cellobiose déshydrogénase (CDH) modifiée ou son domaine flavodéshydrogénase fonctionnel comportant une substitution à l'acide aminé correspondant à N721 de SEQ ID NO: 5 (CDH de *M. thermophilum*) par la glutamine, l'isoleucine, la thréonine ou la leucine, ou une substitution de l'asparagine dans le motif central actif possédant la séquence d'acides aminés NHW par la glutamine, l'isoleucine, la thréonine ou la leucine,
comprenant un domaine flavodéshydrogénase modifié basé sur un des domaines flavodéshydrogénase non modifiés selon les acides aminés 253-831 de SEQ ID NO: 1, les acides aminés 269-845 de SEQ ID NO: 2, les acides aminés 249-787 de SEQ ID NO: 3, les acides aminés 253-829 de SEQ ID NO: 4, les acides aminés 251-828 de SEQ ID NO: 5, les acides aminés 251-829 de SEQ ID NO: 6, les acides aminés 233-771 de SEQ ID NO: 7, les acides aminés 236-774 de SEQ ID NO: 8, les acides aminés 235-773 de SEQ ID NO: 9, les acides aminés 230-768 de SEQ ID NO: 10 ; ledit domaine flavodéshydrogénase modifié possédant une séquence présentant au moins 50 % d'identité de séquence avec un desdits domaines flavodéshydrogénase non modifiés et comprenant en outre la mutation de substitution au niveau de l'acide aminé correspondant à N721 de SEQ ID NO: 5 ou au niveau du motif NHW.

2. CDH modifiée ou son domaine flavodéshydrogénase fonctionnel selon la revendication 1, où le motif central actif possède la séquence X₁X₂NHWX₃X₄X₅, dans laquelle X₁ est un acide aminé quelconque, de préférence sélectionné parmi N, C et R ; X₂ est sélectionné parmi S et A ; X₃ est sélectionné parmi V, M et I ; X₄ est un acide aminé quelconque, de préférence sélectionné parmi G et S ; et X₅ est un acide aminé quelconque, de préférence sélectionné parmi S, A et T ; où, de manière particulièrement préférée, X₄ et X₅ ne sont pas tous deux S.

3. CDH modifiée ou son domaine flavodéshydrogénase fonctionnel selon la revendication 1 ou 2, caractérisé(e) en ce que la CDH est de *dikaryota,* de préférence sélectionnée parmi *ascomycota* et *basidiomycota.*

4. CDH modifiée ou son domaine flavodéshydrogénase fonctionnel selon la revendication 3, caractérisé(e) en ce que la CDH est une CDH modifiée d'une CDH de *Myriococcum thermophilum, Corynascus thermophilus, Chaetomium atrobrunneum (Myceliophthora fergusii), Hypoxylon heamatostroma, Neurospora crassa* ou *Stachybotrys bisby, Athelia rolfsii, Gelatoporia subvermispora, Phanerochaete chrysosporium, Trametes versicolor.*

5. CDH modifiée ou son domaine flavodéshydrogénase fonctionnel selon l'une quelconque des revendications 1 à 4, où ledit domaine flavodéshydrogénase modifié possède une séquence présentant au moins 70 %, de préférence au moins 75 %, au moins 80 %, au moins 85 %, au moins 90 %, au moins 95 %, au moins 98 %, de manière particulièrement préférée au moins 99 % d'identité de séquence avec un desdits domaines flavodéshydrogénase non modifiés et comprend en outre la mutation de substitution à l'acide aminé correspondant à N721 de SEQ ID NO: 5 ou au niveau du motif NHW.

6. CDH modifiée ou son domaine flavodéshydrogénase fonctionnel selon l'une quelconque des revendications 1 à 5, où l'acide aminé correspondant à N721 de SEQ ID NO: 5 ou l'asparagine au niveau du motif NHW est l'acide aminé N723 de SEQ ID NO: 1, N738 de SEQ ID NO: 2, N718 de SEQ ID NO: 3, N722 de SEQ ID NO: 4, N721 de SEQ ID NO: 5, N721 de SEQ ID NO: 6, N704 de SEQ ID NO: 7, N707 de SEQ ID NO: 8, N706 de SEQ ID NO: 9, N701 de SEQ ID NO: 10.

7. CDH modifiée ou son domaine flavodéshydrogénase fonctionnel selon l'une quelconque des revendications 1 à 6, qui est produit(e) de manière recombinante par *Pichia pastoris,* est isolée par diafiltration, chromatographie échangeuse d'ions, et de préférence est en outre purifiée par une chromatographie d'interaction hydrophobe.

8. CDH modifiée ou son domaine flavodéshydrogénase fonctionnel selon l'une quelconque des revendications 1 à 7, caractérisé(e) en ce que la valeur K_{M} de la cellobiose déshydrogénase ou son domaine flavodéshydrogénase fonctionnel pour une réaction d'oxydation du lactose est inférieure à 10 mM, de préférence tel que déterminé avec la CDH ou ledit domaine qui est immobilisé(e) sur une électrode.

9. CDH modifiée ou son domaine flavodéshydrogénase fonctionnel selon l'une quelconque des revendications 1 à 8, caractérisé(e) en ce que la valeur K_{M} de la cellobiose déshydrogénase ou son domaine flavodéshydrogénase fonctionnel pour une réaction d'oxydation du glucose est supérieure à 3 M, de préférence tel que déterminé avec la CDH ou ledit domaine qui est immobilisé(e) sur une électrode.

10. CDH modifiée ou son domaine flavodéshydrogénase fonctionnel selon l'une quelconque des revendications 1 à 9, caractérisé(e) en ce que la valeur K_{M} de la cellobiose déshydrogénase ou son domaine flavodéshydrogénase fonctionnel pour une réaction d'oxydation du galactose est supérieure à 3 M, de préférence tel que déterminé avec la CDH ou ledit domaine qui est immobilisé(e) sur une électrode.

11. Molécule d'acide nucléique codant pour une CDH modifiée ou son domaine flavodéshydrogénase fonctionnel selon l'une quelconque des revendications 1 à 10.

12. Procédé de production d'une CDH modifiée ou de son domaine flavodéshydrogénase fonctionnel selon l'une quelconque des revendications 1 à 10, comprenant l'expression recombinante d'une molécule d'acide nucléique selon la revendication 11 dans une cellule hôte.

13. Électrode comprenant une cellobiose déshydrogénase immobilisée ou son domaine flavodéshydrogénase fonctionnel selon l'une quelconque des revendications 1 à 10 ;
de préférence dans laquelle la cellobiose déshydrogénase est immobilisée par une adsorption, une formation de complexe, de manière particulièrement préférée via un segment de liaison de complexation supplémentaire, une liaison covalente ou ionique, et/ou dans laquelle de préférence la cellobiose déshydrogénase immobilisée est réticulée, en particulier par des agents bifonctionnels, afin d'augmenter la stabilité ou l'activité.

14. Procédé de détection ou de quantification du lactose dans un échantillon comprenant l'étape d'oxydation du lactose dans ledit échantillon avec une CDH modifiée ou son domaine flavodéshydrogénase fonctionnel selon l'une quelconque des revendications 1 à 10, ou une électrode selon la revendication 13, et la détection ou la quantification de ladite oxydation, de préférence où ledit échantillon comprend ou est suspecté de comprendre du glucose et/ou du galactose, de manière particulièrement préférée un échantillon contenant du lait ou un produit laitier.

15. Kit de dosage du lactose comprenant la cellobiose déshydrogénase modifiée ou son domaine flavodéshydrogénase fonctionnel selon l'une quelconque des revendications 1 à 10 ou une électrode selon la revendication 13 et un moyen de maintien de l'échantillon et/ou des étalons du lactose.
